# EUROPEAN PATENT APPLICATION

(11) **EP 4 406 945 A1**
(43) Date of publication of application: **31.07.2024**
(21) Application number: 23382069.5
(22) Date of filing: 27.01.2023
(51) Int. Cl.: C07D 309/04, C07D 251/34, C07D 405/12, A61K 31/166, A61K 31/351, A61P 31/04

(54) **NEW COMPOUNDS FOR TREATING STREPTOCOCCUS INFECTIONS**

(71) Applicant: ABAC THERAPEUTICS, S.L., 08950 Esplugues de Llobregat (Barcelona) (ES)
(72) Inventor: TORRENS JOVER, Antoni, 08021 Terrassa (Barcelona) (ES); GARGALLO VIOLA, Domingo, 08860 Castelldefels (Barcelona) (ES); CAAMAÑO MOURE, Ana María, 15707 Santiago de Compostela (A Coruña) (ES); LLORENTE FERNÁNDEZ, Ana Virginia, 08902 Hospitalet de Llobregat (Barcelona) (ES)
(74) Representative: Herrero & Asociados, S.L.

(57) **Abstract**

The present invention relates to new bactericidal compounds of formula (I) useful against *Streptococcus sp.* infections, especially against infections of *Streptococcus pneumoniae, Streptococcus pyogenes and Streptococcus agalactiae.* The invention is also directed to the process for the preparation of said bactericidal compounds, to compositions comprising them, and to their use as medicaments.

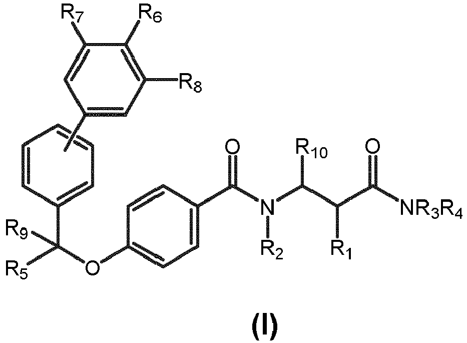

## Description

### FIELD OF THE INVENTION

The present invention relates to new bactericidal compounds useful against *Streptococcus sp.* infections, especially against infections of *Streptococcus pneumoniae, Streptococcus pyogenes and Streptococcus agalactiae.* The invention is also directed to the process for the preparation of said bactericidal compounds, to compositions comprising them, and to their use as medicaments.

### BACKGROUND OF THE INVENTION

Infectious diseases represent a significant global burden affecting society. Most of these diseases are due to exposure to or the invasion of host cells and organs by microorganisms. These pathogens disrupt the normal function of the human body by hindering immune responses and producing harmful toxins. Infectious diseases can easily spread from person-to-person *via* contact with body fluids, indirect contact or through animal vectors such as mosquitoes and ticks. Common widespread diseases of the respiratory system occur when microorganisms invade the respiratory tract. Infectious respiratory diseases are globally seen as a major health concern because they can rapidly become severe and lead to death. Respiratory diseases are categorized into upper and lower respiratory tract infection, which are more severe because pathogens infect sterile parts of the respiratory system such as the lungs, trachea, and bronchi. Globally, approximately 2.49 million deaths resulted from lower respiratory tract infections in 2019, making it the fourth leading cause of deaths for all ages and thus they remain a healthcare concern for specific high-risk populations.

*Streptococcus pneumoniae,* a Gram-positive bacterium, also known as pneumococcus, is an infectious pathogen responsible for millions of deaths worldwide. Diseases caused by this bacterium are classified as pneumococcal diseases. This pathogen colonizes the nasopharynx of its host asymptomatically, but overtime can migrate to sterile tissues and organs and cause infections. It can survive in both aerobic and anaerobic conditions.

*Streptococcus pyogenes* is the causative agent in a wide range of group A streptococcal infections (GAS). These infections may be non-invasive or invasive. The noninvasive infections tend to be more common and less severe. The most common of these infections include streptococcal pharyngitis (strep throat) and impetigo. Scarlet fever is another example of Group A non-invasive infection. The invasive infections caused by group A beta-haemolytic streptococci tend to be more severe and less common. This occurs when the bacterium can infect areas where it is not usually found, such as the blood and the organs. The diseases that may be caused include streptococcal toxic shock syndrome, necrotizing fasciitis, pneumonia, and bacteraemia. Globally, GAS has been estimated to cause more than 500,000 deaths every year, making it one of the world's leading pathogens.

*Streptococcus agalactiae*, or group B streptococcus, GBS, causes pneumonia and meningitis in new-borns and the elderly, with occasional systemic bacteraemia. Importantly, Streptococcus agalactiae is the most common cause of meningitis in infants from one month to three months old. They can also colonize the intestines and the female reproductive tract, increasing the risk for premature rupture of membranes during pregnancy, and transmission of the organism to the infant.

Pneumonia is currently the most common pneumococcal disease. Pneumococcal pneumonia is a global health concern and vastly affects children under the age of five as well as the elderly and individuals with pre-existing health conditions. *Streptococcus pneumoniae* has a large selection of virulence factors that promote adherence, invasion of host tissues, and allows it to escape host immune defences. It is the main microorganism that causes community-acquired pneumonia. It is considered the 4^{th} agent in number of deaths in the world. Worldwide pneumonia is the leading cause of death in children under the age of five. The World Health Organization reported that a child dies from pneumonia every 20 seconds. There are approximately 900,000 cases of pneumococcal pneumonia that occur annually within the US. In addition, United Nations Children Fund stated that in 2016, pneumonia accounted for 16% of the fatalities observed among young children under the age of five worldwide. Pneumococcal pneumonia leads to about 300,000-600,000 elderly hospitalizations annually in the US, and the elderly have reduced survival rates. There are different types of pneumonia: community-acquired pneumonia (CAP), atypical pneumonia, hospital acquired pneumonia, and aspiration pneumonia. These differ based on where someone contracts the infection and what bacteria cause the disease. Currently, the most common form of pneumonia is CAP (which is mostly pneumococcal). This type of pneumonia spreads *via* person-to-person contacts in the community, but outside of healthcare facilities, by breathing in aerosol droplets from a carrier or infected person. Worldwide, CAP is currently the leading cause of death for young children who are under the age of five. Infants, young children, the elderly, smokers, and immunocompromised individuals are all at a higher risk of developing pneumonia due to a weakened immune system. CAP has a higher occurrence rate in the elderly compared to younger populations and is also the fifth leading cause of death in the elderly population.

With diagnostic methods improving, pneumococcal disease treatments are also being updated. Antibiotics are available to reduce the colonization of *Streptococcus pneumoniae*, however, the efficacy of antibiotics is being reduced due to the increase in antibiotic resistance. Broad-spectrum antibiotics are no longer as effective. Traditionally, β-lactams and macrolide antimicrobials have been the principal therapeutic options for pneumococcal infections. However, the emergence of pneumococci with reduced susceptibilities to these agents has emphasized the need for alternative therapies having high efficacy against respiratory pathogens, including resistant strains, and a low potential for resistance induction.

One of the alternatives studied is the use of combinations of known antibiotics in the hope of a synergistic effect on resistant bacterial strains. However, this approach shows limitations in the choice of possible combinations and the long-term applicability of this strategy.

A second strategy aims to increase the sensitivity of bacterial strains to known antibiotics.

A third strategy consists in discovering new compounds capable of selectively controlling the bacteria *Streptococcus pneumoniae.* This approach has several advantages compared to the use of broad-spectrum antibiotics, because selective agents allow to kill or inhibit only those bacteria species that are causing the disease (i.e., *Streptococcus pneumoniae*). As such, it leaves most of the beneficial bacteria unaffected, hence minimizing the collateral damage on the microbiota. Moreover, they have low propensity for bacterial resistance development.

For all these reasons, there is a real unmet need to find new selective compounds active against *Streptococcus pneumoniae* that allow to treat the disease effectively, avoiding the disadvantages of the use of broad-spectrum agents.

### SUMMARY OF THE INVENTION

The present invention discloses novel compounds with a potent antibiotic activity against *Streptococcus sp.* and more particularly against *Streptococcus pneumoniae Streptococcus pyogenes* and *Streptococcus agalactiae.* The compounds of the invention can be useful for the treatment of infections caused by these pathogens.

The invention is directed in a main aspect to a compound of Formula (I), 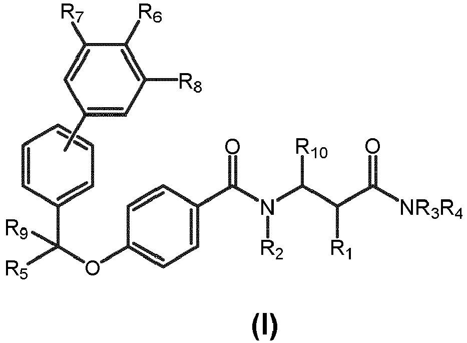 wherein **R₁, R₂, R₃, R₄, R₅, R₆, R₇, R₈, R₉** and **R₁₀** are as defined below in the detailed description.

A further aspect of the invention refers to the processes for preparation of compounds of formula (I).

It is also an aspect of the invention a pharmaceutical composition comprising a compound of formula (I).

Finally, it is an aspect of the invention a compound of formula (I) for use in therapy and more particularly for the treatment of bacterial infections caused *Streptococcus sp,* particularly by *Streptococcus pneumoniae, Streptococcus pyogenes and Streptococcus agalactiae.*

### DETAILED DESCRIPTION OF THE INVENTION

The invention is directed to a family of compounds, in particular, to showing a potent bactericidal activity against *Streptococcus sp* thus, solving the above problem of identifying alternative compounds selectively controlling *Streptococcus* but avoiding the disadvantages of having a broad-spectrum.

The applicant has found that the problem of providing a new effective and alternative solution for preventing and treating *Streptococcus* infections can surprisingly be solved by using compounds of the present inventions.

In a first aspect, the present invention is directed to a compound of formula (I): 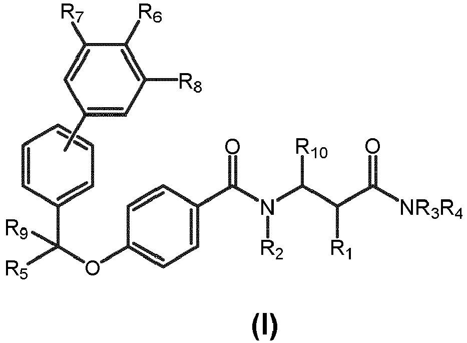 wherein:
R₁ is a hydrogen atom, a halogen atom, a branched or unbranched C₁₋₆ alkyl radical or - OR₁ₐ;
   **R₁ₐ** is a hydrogen atom or a branched or unbranched C₁₋₆ alkyl radical;
**R₂** is a hydrogen atom or a branched or unbranched C₁₋₆ alkyl radical;
**R₃** is a hydrogen atom, a branched or unbranched C₁₋₆ alkyl, or -(CH₂)ₘ-O**R₃ₐ**;
   **R₃ₐ** is a hydrogen atom or a branched or unbranched C₁₋₆ alkyl radical;
**R₄** is a hydrogen atom, a branched or unbranched C₁₋₆ alkyl, or -(CH₂)ₙ-O**R₄ₐ**;
   **R₄ₐ** is a hydrogen atom or a branched or unbranched C₁₋₆ alkyl radical;
**R₅** is a hydrogen atom; a branched or unbranched C₁₋₆ alkyl radical optionally substituted; a halogen atom; a -CN; a C₁₋₆ haloalkyl radical; an optionally substituted -(CH₂)ᵣ-C₃₋₉ cycloalkyl; an optionally substituted -(CH₂)ₛ-C₃₋₉ heterocycloalkyl containing at least one heteroatom selected from N, O or S; an optionally substituted -(CH₂)ⱼ-aryl; an optionally substituted -(CH₂)ᵢ-heteroaryl containing at least one heteroatom selected from N, O or S; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, -OR₅ₐ, -NR₅ₐR_{5b}, NR₅ₐC(O)R_{5b}, -NR₅ₐC(O)NR_{5b}R_{5c}, -C(O)OR₅ₐ, -C(O)NR₅ₐR_{5b}, -OCH₂CH₂OH and - C(CH₃)₂OR₅ₐ;
   **R₅ₐ**, **R_{5b}** and **R_{5c}** are independently selected from a hydrogen atom or branched or unbranched C₁₋₆ alkyl radical;
**R₆, R₇** and **R₈** are independently from one another selected from a hydrogen atom, a branched or unbranched C₁₋₆ alkyl radical optionally substituted; a halogen atom; a -CN; a C₁₋₆ haloalkyl radical; an optionally substituted C₃₋₉ cycloalkyl; an optionally substituted C₃₋₉ heterocycloalkyl containing at least one heteroatom selected from N, O or S; an -O-C₁₋₆-alkyl optionally substituted; an -O-C₁₋₆-haloalkyl; an optionally substituted -O-C₃₋₉ cycloalkyl; an optionally substituted -O-C₃₋₉ heterocycloalkyl containing at least one heteroatom selected from N, O or S; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, , -ORₐ, -NRₐR_{b}, NRₐC(O)R_{b}, -C(O)ORₐ, -C(O)NRₐR_{b}, -OCH₂CH₂OH, - C(CH₃)₂ORₐ;
   **Rₐ,** and **R_{b}** are independently selected from a hydrogen atom or branched or unbranched C₁₋₆ alkyl radical
**R₉** is a hydrogen atom or a branched or unbranched C₁₋₆ alkyl radical;
**R₁₀** is a hydrogen atom; a branched or unbranched C₁₋₆ alkyl radical optionally substituted; a -CN; a C₁₋₆ haloalkyl radical; an optionally substituted -(CH₂)ᵣ-C₃₋₉ cycloalkyl; an optionally substituted -(CH₂)ⱼ-aryl; an -O-C₁₋₆-alkyl optionally substituted; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, - -OR₁₀ₐ, -NR₁₀ₐR_{10b}, NR₁₀ₐC(O)R_{10b}, -NR₁₀ₐC(O)NR_{10b}R_{10c}, -C(O)OR₁₀ₐ, -C(O)NR₁₀ₐR_{10b}, -OCH₂CH₂OH, - NR₁₀ₐS(O)₂NR_{10b}R_{10c}, -C(CH₃)₂OR₁₀ₐ;
   **R₁₀ₐ, R_{10b}** and **R_{10c}** are independently selected from a hydrogen atom or branched or unbranched C₁₋₆ alkyl radical;
**n** is 0, 1, 2 or 3;
**m** is 0, 1, 2 or 3;
**r** is 0, 1, 2 or 3;
**s** is 0, 1, 2 or 3
**j** is 0, 1, 2 or 3;
**i** is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The compounds of general formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

For the sake of clarity, the expression like "a compound according to formula (I), wherein R₁, R₂ and R₃ are as defined below in the detailed description" would (just like the expression "a compound of formula (I) as defined in the claims) refer to "a compound according to formula (I)", wherein the definitions of the respective substituents R₁ etc. (also from the cited claims) are applied.

For clarity purposes, all groups and definitions described in the present description and referring to compounds of formula (I), also apply to all intermediates of synthesis.

"Halogen" or "halo" as referred in the present invention represent fluorine, chlorine, bromine or iodine. When the term "halo" is combined with other substituents, such as for instance "C₁₋₆ haloalkyl" or "C₁₋₆ haloalkoxy" it means that the alkyl or alkoxy radical can respectively contain at least one halogen atom.

"C₁₋₆ alkyl", as referred to in the present invention, are saturated aliphatic radicals. They may be unbranched (linear) or branched and are optionally substituted. C₁₋₆-alkyl as expressed in the present invention means an alkyl radical of 1, 2, 3, 4, 5 or 6 carbon atoms. Preferred alkyl radicals according to the present invention include but are not restricted to methyl, ethyl, propyl, n-propyl, isopropyl, butyl, n-butyl, tert-butyl, isobutyl, sec-butyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl. The most preferred alkyl radical are C₁₋₄ alkyl, such as methyl, ethyl, propyl, n-propyl, isopropyl, butyl, n-butyl, tert-butyl, isobutyl, sec-butyl, 1-methylpropyl, 2-methylpropyl or 1,1-dimethylethyl. Alkyl radicals, as defined in the present invention, are optionally mono- or polysubstituted by substituents independently selected among others from a halogen, branched or unbranched C₁₋₆-alkoxy, branched or unbranched C₁₋₆-alkyl, C₁₋₆₋haloalcoxy, C₁₋₆-haloalkyl, trihaloalkyl or a hydroxyl group.

"C₁₋₆ alkoxy" as referred to in the present invention, is understood as meaning an alkyl radical as defined above attached via oxygen linkage to the rest of the molecule. Examples of alkoxy include, but are not limited to methoxy, ethoxy, propoxy, butoxy or tert-butoxy.

"C₃₋₆ Cycloalkyl" as referred to in the present invention, is understood as meaning saturated and unsaturated (but not aromatic), cyclic hydrocarbons having from 3 to 6 carbon atoms which can optionally be unsubstituted, mono- or polysubstituted.

Examples for cycloalkyl radical preferably include but are not restricted to cyclopropyl, cyclobutyl, cyclopentyl, cyclohexyl. Cycloalkyl radicals, as defined in the present invention, are optionally mono- or polysubstituted by substituents independently selected among others from a halogen atom, branched or unbranched C₁₋₆-alkyl, branched or unbranched C₁₋₆-alkoxy, C₁₋₆₋haloalcoxy, C₁₋₆-haloalkyl, trihaloalkyl or a hydroxyl group.

A heterocyclyl radical or group (also called heterocyclyl hereinafter) is understood as meaning 4 to 18 membered mono or fused polycyclic heterocyclic ring systems, with at least one saturated or unsaturated ring which contains one or more heteroatoms selected from the group consisting of nitrogen, oxygen and/or sulfur in the ring. A heterocyclic group can also be substituted once or several times.

Subgroups inside the heterocyclyls as understood herein include heteroaryls and non-aromatic heterocyclyls.
- the heteroaryl (being equivalent to heteroaromatic radicals or aromatic heterocyclyls) is an aromatic 5 to 18 membered mono or fused polycyclic heterocyclic ring system of one or more rings of which at least one aromatic ring contains one or more heteroatoms from the group consisting of nitrogen, oxygen and/or sulfur in the ring; preferably it is a 5 to 18 membered mono or fused polycyclic aromatic heterocyclic ring system of one or two rings of which at least one aromatic ring contains one or more heteroatoms selected from the group consisting of nitrogen, oxygen and/or sulfur in the ring; more preferably it is selected from furan, benzofuran, thiophene, benzothiophene, pyrrole, pyridine, pyrimidine, pyrazine, quinoline, isoquinoline, phthalazine, benzothiazole, indole, benzotriazole, carbazole, quinazoline, thiazole, imidazole, pyrazole, oxazole, oxadiazole, thiophene and benzimidazole;
- the non-aromatic heterocyclyl is a 4 to 18 membered mono or fused polycyclic heterocyclic ring system of one or more rings of which at least one ring - with this (or these) ring(s) then not being aromatic - contains one or more heteroatoms from the group consisting of nitrogen, oxygen and/or sulfur in the ring; preferably it is a 4 to 18 membered mono or fused polycyclic heterocyclic ring system of one or two rings of which one or both rings - with this one or two rings then not being aromatic - contain/s one or more heteroatoms selected from the group consisting of nitrogen, oxygen and/or sulfur in the ring, more preferably it is selected from azetidine, oxetane, tetrahydrofuran, oxazepam, pyrrolidine, piperidine, piperazine, tetrahydropyran, morpholine, indoline, oxopyrrolidine, benzodioxane, especially is piperazine, benzodioxane, morpholine, tetrahydropyran, piperidine, oxopyrrolidine and pyrrolidine.

Preferably, in the context of this invention heterocyclyl is defined as a 4 to 18 membered mono or fused polycyclic ring system of one or more saturated or unsaturated rings of which at least one ring contains one or more heteroatoms selected from the group consisting of nitrogen, oxygen and/or sulfur in the ring. Preferably it is a 4 to 18 membered mono or fused polycyclic heterocyclic ring system of one or two saturated or unsaturated rings of which at least one ring contains one or more heteroatoms selected from the group consisting of nitrogen, oxygen, and sulfur in the ring. More preferably, it is a 4 to 12 membered mono or bicyclic heterocyclyl ring system containing one nitrogen atom and optionally a second heteroatom selected from nitrogen and oxygen. In another preferred embodiment of the invention, said heterocyclyl is a substituted mono or bicyclic heterocyclyl ring system.

Preferred examples of heterocyclyls include azetidine, azepane, oxetane, tetrahydrofuran, oxazepam, pyrrolidine, imidazole, oxadiazole, tetrazole, pyridine, pyrimidine, piperidine, piperazine, benzofuran, benzimidazole, indazole, benzodiazole, thiazole, benzothiazole, tetrahydropyran, morpholine, indoline, furan, triazole, isoxazole, pyrazole, thiophene, benzothiophene, pyrrole, pyrazine, pyrrolo[2,3b]pyridine, quinoline, isoquinoline, tetrahydroisoquinoline, phthalazine, benzo-1,2,5-thiadiazole, indole, benzotriazole, benzoxazole oxopyrrolidine, pyrimidine, benzodioxolane, benzodioxane, carbazole and quinazoline, 3,9-diazaspiro[5.5]undecane, 2,8-diazaspiro[4.5]decane, 2,7-diazaspiro[3.5]nonane, 2,7-diazaspiro[4.4]nonane, octahydropyrrolo[3,4-c]pyrrole, especially is pyridine, piperazine, pyrazine, indazole, benzodioxane, thiazole, benzothiazole, morpholine, tetrahydropyran, pyrazole, imidazole, piperidine, thiophene, indole, benzimidazole, pyrrolo[2,3-b]pyridine, benzoxazole, oxopyrrolidine, pyrimidine, oxazepane, pyrrolidine, azetidine, azepane, oxetane, tetrahydrofuran, 3,9-diazaspiro[5.5]undecane, 2,8-diazaspiro[4.5]decane and 2,7-diazaspiro[3.5]nonane.

An *N*-containing heterocyclyl is a heterocyclic ring system of one or more saturated or unsaturated rings of which at least one ring contains a nitrogen and optionally one or more further heteroatoms selected from the group consisting of nitrogen, oxygen and/or sulfur in the ring; preferably is a heterocyclic ring system of one or two saturated or unsaturated rings of which at least one ring contains a nitrogen and optionally one or more further heteroatoms selected from the group consisting of nitrogen, oxygen and/or sulfur in the ring, more preferably is selected from azetidine, azepane, oxazepam, pyrrolidine, imidazole, oxadiazole, tetrazole, azetidine, pyridine, pyrimidine, piperidine, piperazine, benzimidazole, indazole, benzothiazole, benzodiazole, morpholine, indoline, triazole, isoxazole, pyrazole, pyrrole, pyrazine, pyrrolo[2,3-b]pyridine, quinoline, quinolone, isoquinoline, tetrahydrothienopyridine, phthalazine, benzo-1,2,5-thiadiazole, indole, benzotriazole, benzoxazole oxopyrrolidine, carbazole, thiazole, 3,9-diazaspiro[5.5]undecane, 2,8-diazaspiro[4.5]decane, 2,7-diazaspiro[3.5]nonane, 2,7-diazaspiro[4.4]nonane or octahydropyrrolo[3,4-c]pyrrole.

In connection with aromatic heterocyclyls (heteroaryls), non-aromatic heterocyclyls, aryls and cycloalkyls, when a ring system falls within two or more of the above cycle definitions simultaneously, then the ring system is defined first as an aromatic heterocyclyl (heteroaryl) if at least one aromatic ring contains a heteroatom. If no aromatic ring contains a heteroatom, then the ring system is defined as a non-aromatic heterocyclyl if at least one non-aromatic ring contains a heteroatom. If no non-aromatic ring contains a heteroatom, then the ring system is defined as an aryl if it contains at least one aryl cycle. If no aryl is present, then the ring system is defined as a cycloalkyl if at least one non-aromatic cyclic hydrocarbon is present.

"Heterocycloalkyl" as referred to in the present invention, are understood as meaning saturated and unsaturated (but not aromatic), generally 5 or 6 membered cyclic hydrocarbons which can optionally be unsubstituted, mono- or polysubstituted and which have at least one heteroatom in their structure selected from N, O or S. Examples for heterocycloalkyl radical preferably include but are not restricted to pyrroline, pyrrolidine, pyrazoline, aziridine, azetidine, tetrahydropyrrole, oxirane, oxetane, dioxetane, tetrahydropyrane, tetrahydrofurane, dioxane, dioxolane, oxazolidine, piperidine, piperazine, morpholine, azepane or diazepane. Heterocycloalkyl radicals, as defined in the present invention, are optionally mono- or polysubstituted by substitutents independently selected from a halogen atom, branched or unbranched C₁₋₆-alkyl, branched or unbranched C₁₋₆-alkoxy, C₁₋₆-haloalkoxy, C₁₋₆-haloalkyl, trihaloalkyl or a hydroxyl group. More preferably heterocycloalkyl in the context of the present invention are 5 or 6-membered ring systems optionally at least monosubstituted.

"Aryl" as referred to in the present invention, is understood as meaning ring systems with at least one aromatic ring but without heteroatoms even in only one of the rings. These aryl radicals may optionally be mono-or polysubstituted by substitutents independently selected from a halogen atom, -CN, branched or unbranched C₁₋₆-alkyl, branched or unbranched C₁₋₆-alkoxy, C₁₋₆₋haloalcoxy, C₁₋₆-haloalkyl, a heterocyclyl group and a hydroxyl group. Preferred examples of aryl radicals include but are not restricted to phenyl, naphthyl, fluoranthenyl, fluorenyl, tetralinyl, indanyl or anthracenyl radicals, which may optionally be mono- or polysubstituted, if not defined otherwise. More preferably aryl in the context of the present invention is a 6-membered ring system optionally at least mono or polysubstituted.

"Heteroaryl" as referred to in the present invention, is understood as meaning heterocyclic ring systems which have at least one aromatic ring and contain one or more heteroatoms from the group consisting of N, O or S and may optionally be mono-or polysubstituted by substituents independently selected from a halogen atom, branched or unbranched C₁₋₆-alkyl, branched or unbranched C₁₋₆-alkoxy, C₁₋₆-haloalkoxy, C₁₋₆-haloalkyl trihaloalkyl or a hydroxyl group. Preferred examples of heteroaryls include but are not restricted to furan, benzofuran, pyrrole, pyridine, pyrimidine, pyridazine, pyrazine, quinoline, isoquinoline, phthalazine, triazole, pyrazole, isoxazole, indole, benzotriazole, benzodioxolane, benzodioxane, benzimidazole, carbazole and quinazoline. More preferably heteroaryl in the context of the present invention are 5 or 6-membered ring systems optionally at least monosubstituted.

The term "condensed" according to the present invention means that a ring or ring-system is attached to another ring or ring-system, whereby the terms "annulated" or "annelated" are also used by those skilled in the art to designate this kind of attachment.

The term "ring system" according to the present invention refers to a system consisting of at least one ring of connected atoms but also including systems in which two or more rings of connected atoms are joined with "joined" meaning that the respective rings are sharing one (like a spiro structure), two or more atoms being a member or members of both joined rings. The "ring system" thus defined comprises saturated, unsaturated, or aromatic carbocyclic rings which contain optionally at least one heteroatom as ring member and which are optionally at least mono-substituted and may be joined to other carbocyclic ring systems such as aryl radicals, heteroaryl radicals, cycloalkyl radicals etc.

The terms "condensed", "annulated" or "annelated" are also used by those skilled in the art to designate this kind of join.

A "leaving group" is a group that in a heterolytic bond cleavage keeps the electron pair of the bond. Suitable leaving groups are well known in the art and include C!, Br, I and - O-SO₂R¹⁴, wherein R¹⁴ is F, C₁₋₄-alkyl, C₁₋₄-haloalkyl, or optionally substituted phenyl. The preferred leaving groups are Cl, Br, I, tosylate, mesylate, triflate, nonaflate and fluorosulphonate.

"Protecting group" is a group that is chemically introduced into a molecule to avoid that a certain functional group from that molecule undesirably reacts in a subsequent reaction. Protecting groups are used, among others, to obtain chemoselectivity in chemical reactions. The preferred protecting group in the context of the invention are Boc (tert-butoxycarbonyl) or Teoc (2-(trimethylsilyl)ethoxycarbonyl).

The term "salt" is to be understood as meaning any form of the active compound according to the invention in which this assumes an ionic form or is charged and is coupled with a counter-ion (a cation or anion). The definition particularly includes physiologically acceptable salts, this term must be understood as equivalent to "pharmaceutically acceptable salts".

The term "pharmaceutically acceptable salts" in the context of this invention means any salt that is tolerated physiologically (normally meaning that it is not toxic, particularly as a result of the counter-ion) when used in an appropriate manner for a treatment, particularly applied or used in humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid. In addition, the pharmaceutically acceptable salts may be formed with a physiologically tolerated cation, preferably inorganic, particularly when used on humans and/or mammals. Salts with alkali and alkali earth metals are particularly preferred, as well as those formed with ammonium cations (NH₄⁺).Preferred salts are those formed with (mono) or (di)sodium, (mono) or (di)potassium, magnesium or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals.

The compounds of the invention may be present in crystalline form or in amorphous form.

Any compound that is a solvate of a compound according to formula (I) defined above is understood to be also covered by the scope of the invention. Methods of solvation are generally known within the art. Suitable solvates are pharmaceutically acceptable solvates. The term "solvate" is to be understood as meaning any form of the active compound according to the invention in which this compound has attached to it via noncovalent binding another molecule (most likely a polar solvent) especially including hydrates and alcoholates, like methanolate or ethanolate.

The term "co-crystal" is to be understood as a crystalline material comprising a specific active compound with at least one additional component, usually a co-crystal former, and of which at least two of the constituents are held together by weak interactions. Weak interaction is being defined as an interaction which is neither ionic nor covalent and includes for example: hydrogen bonds, van der Waals forces, and π-π interactions.

The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the compounds of the invention: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Any compound that is a prodrug of a compound of general formula (I) is within the scope of the invention. Particularly favored prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

Any compound that is a *N*-oxide of a compound according to the invention like a compound according to formula (I) defined above is understood to be also covered by the scope of the invention.

The compounds of formula (I) as well as their salts or solvates are preferably in pharmaceutically acceptable or substantially pure form. By pharmaceutically acceptable pure form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts. This applies also to its solvates or prodrugs.

In a particular and preferred embodiment of the invention, **R₁** is a hydrogen atom or - O**R₁ₐ.**

In an even more preferred embodiment **R₁** is a hydrogen atom or -O**R₁ₐ, R₁ₐ** being a hydrogen atom.

In another particular and preferred embodiment of the invention, **R₂** is hydrogen atom.

Yet another particular and preferred embodiment of the invention is that where **R₃** is a hydrogen atom, a methyl group, or -(CH₂)ₘ-O**R₃ₐ.**

In an even more preferred embodiment **R₃** is hydrogen atom, a methyl group, or -(CH₂)ₘ-**OR₃ₐ, m** being 0 or 2 and **R₃ₐ** being a hydrogen atom.

Another particular and preferred embodiment of the invention is that where **R₄** is a hydrogen atom, a methyl group, or -(CH₂)ₙ-O**R₄ₐ.**

In an even more preferred embodiment **R₄** is hydrogen atom, a methyl group, or -(CH₂)ₘ-O**R₄ₐ,** n being 0 or 2 and **R₃ₐ** being a hydrogen atom.

Still another particular and preferred embodiment of the invention is that where **R₅** is a branched or unbranched C₁₋₆ alkyl radical optionally substituted; an optionally substituted -(CH₂)ᵣ-C₃₋₉ cycloalkyl; or an optionally substituted -(CH₂)ₛ-C₃₋₉ heterocycloalkyl containing at least one heteroatom selected from N, O or S.

In an even more preferred embodiment **R₅** is isobutyl, isopropyl; or one of the following groups:

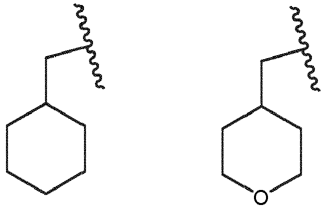

An additional particular and preferred embodiment of the invention is where **R₆, R₇** and **R₈** are independently from one another selected from a hydrogen atom, a branched or unbranched C₁₋₆ alkyl radical optionally substituted; a halogen atom; or a C₁₋₆ haloalkyl radical.

In an even more preferred embodiment **R₆, R₇** and **R₈** are independently from one another selected from a hydrogen atom, a F; or a trifluoromethyl.

Another particular embodiment of the invention is that where **R₉** is a hydrogen atom.

In a still particular and preferred embodiment, **R₁₀** is a hydrogen atom or a branched or unbranched C₁₋₆ alkyl radical optionally substituted.

In an even more preferred embodiment **R₁₀** is a hydrogen atom or a methyl.

A further particular and preferred embodiment of the invention comprises a compound of formula (I): 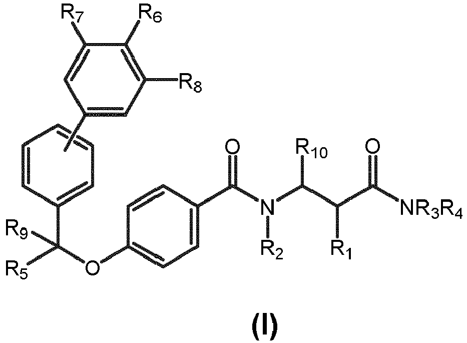 wherein:
**R₁** is a hydrogen atom or -O**R₁ₐ**;
   **R₁ₐ** is a hydrogen atom;
**R₂** is a hydrogen atom;
**R₃** is a hydrogen atom, a branched or unbranched C₁₋₆ alkyl, preferably methyl, or - (CH₂)ₘ-O**R₃ₐ;**
   **R₃ₐ** is a hydrogen atom;
**R₄** is a hydrogen atom, a branched or unbranched C₁₋₆ alkyl, preferably methyl, or -(CH₂)ₙ-**OR₄ₐ;**
   **R₄ₐ** is a hydrogen atom;
**R₅** is a branched or unbranched C₁₋₆ alkyl radical optionally substituted; an optionally substituted -(CH₂)ᵣ-C₃₋₉ cycloalkyl; or an optionally substituted -(CH₂)ₛ-C₃₋₉ heterocycloalkyl containing at least one heteroatom selected from N, O or S.
**R₆, R₇** and **R₈** are independently from one another selected from a hydrogen atom, a branched or unbranched C₁₋₆ alkyl radical optionally substituted; a halogen atom; or a C₁₋₆ haloalkyl radical;
**R₉** is a hydrogen atom or a branched or unbranched C₁₋₆ alkyl radical;
**R₁₀** is is a hydrogen atom or a branched or unbranched C₁₋₆ alkyl radical optionally substituted;
**n** is 0, 1, 2 or 3;
**m** is 0, 1, 2 or 3;
**r** is 0, 1, 2 or 3;
**s** is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

A stil more particular and preferred embodiment of the invention comprises a compound of formula (I): 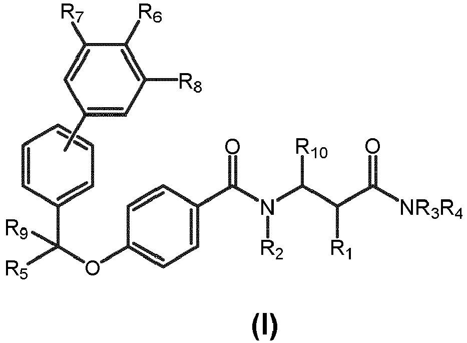 wherein:
**R₁** is a hydrogen atom or -O**R₁ₐ**;
   **R₁ₐ** is a hydrogen atom;
**R₂** is a hydrogen atom;
**R₃** is a hydrogen atom, a branched or unbranched C₁₋₆ alkyl, preferably methyl, or - (CH₂)ₘ-O**R₃ₐ;**
   **R₃ₐ** is a hydrogen atom;
**R₄** is a hydrogen atom, a branched or unbranched C₁₋₆ alkyl, preferably methyl, or-(CH₂)ₙ-**OR₄ₐ;**
   **R₄ₐ** is a hydrogen atom;
**R₅** is isobutyl, isopropyl; or one of the following groups: 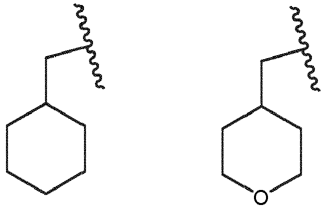
**R₆, R₇** and **R₈** are independently from one another selected from a hydrogen atom, a F; or a trifluoromethyl.
**R₉** is a hydrogen atom;
**R₁₀** is a hydrogen atom or a methyl;
**n** is 0, 1, 2 or 3;
**m** is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

A particularly preferred embodiment of the invention is represented by compounds of formula (I) having subformulas **(Ia)** or **(Ib)**: 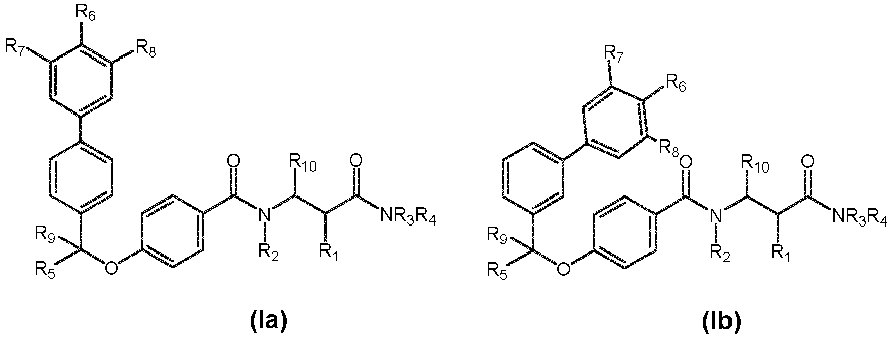 wherein **R₁, R₂, R₃, R₄, R₅, R₆, R₇, R₈, R₉** and **R₁₀** are as defined along the detailed description and claims.

A still more preferred embodiment is represented by compounds with the general formula **(Ia₁)** or **(Ib₁):** 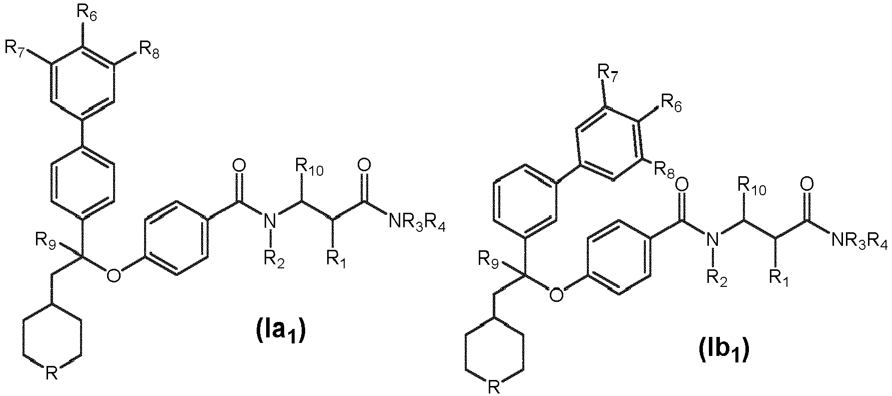 wherein **R₁, R₂, R₃, R₄, R₆, R₇, R₈, R₉** and **R₁₀** are as defined along the detailed description and claims and **R** represents -CH₂- or -O-.

The compounds of the present invention represented by the above described formula (I), (Ia), (Ib), (Ia₁) or (Ib₁) may include enantiomers depending on the presence of chiral centers or isomers depending on the presence of double bonds (e.g. Z, E). The single stereoisomers, enantiomers or diastereoisomers and mixtures thereof fall within the scope of the present invention.

The preferred compounds of the invention are selected from:
**[1]** *N*-((S)-3-Amino-2-hydroxy-3-oxopropyl)-4-((S)-2-cyclohexyl-1-(4'-fluoro-[1,1'-biphenyl]-4-yl)ethoxy)benzamide;
**[2]** *N*-((S)-3-Amino-2-hydroxy-3-oxopropyl)-4-((S)-2-cyclohexyl-1-(4'-(trifluoromethyl)-[1,1'-biphenyl]-3-yl)ethoxy)benzamide;
**[3]** *N*-((S)-3-Amino-2-hydroxy-3-oxopropyl)-4-((S)-2-cyclohexyl-1-(4'-(trifluoromethyl)-[1,1'-biphenyl]-4-yl)ethoxy)benzamide;
**[4]** *N*-((S)-3-amino-2-hydroxy-3-oxopropyl)-4-((S)-2-(tetrahydro-2H-pyran-4-yl)-1-(4'-(trifluoromethyl)-[1,1'-biphenyl]-4-yl)ethoxy)benzamide;
**[5]** (N-((S)-3-amino-2-hydroxy-3-oxopropyl)-4-((S)-3-methyl-1-(4'-(trifluoromethyl)-[1,1'-bipheny]-4-yl)butoxy)benzamide;
**[6]** *N*-((S)-3-amino-2-hydroxy-3-oxopropyl)-4-((S)-2-methyl-1-(4'-(trifluoromethyl)-[1,1'-biphenyl]-4-yl)propoxy)benzamide;
**[7]** N-((S)-3-amino-2-hydroxy-3-oxopropyl)-4-((R)-2-cyclohexyl-1-(4'-(trifluoromethyl)-[1,1'-biphenyl]-3-yl)ethoxy)benzamide;
**[8]** 4-((S)-2-cyclohexyl-1-(4'-(trifluoromethyl)-[1,1'-biphenyl]-3-yl)ethoxy)-N-((S)-2-hydroxy-3-(methylamino)-3-oxopropyl)benzamide;
**[9]** 4-((S)-2-cyclohexyl-1-(4'-(trifluoromethyl)-[1,1'-biphenyl]-3-yl)ethoxy)-N-((S)-3-(dimethylamino)-2-hydroxy-3-oxopropyl)benzamide;
**[10]** N-((R)-3-amino-2-hydroxy-3-oxopropyl)-4-((S)-2-cyclohexyl-1-(4'-(trifluoromethyl)-[1,1'-biphenyl]-3-yl)ethoxy)benzamide;
**[11]** N-((R)-3-amino-2-hydroxy-3-oxopropyl)-4-((R)-2-cyclohexyl-1-(4'-(trifluoromethyl)-[1,1'-biphenyl]-3-yl)ethoxy)benzamide;
**[12]** *N*-((R)-4-amino-4-oxobutan-2-yl)-4-((S)-2-cyclohexyl-1-(4'-(trifluoromethyl)-[1,1'-biphenyl]-4-yl)ethoxy)benzamide;
**[13]** 4-((S)-2-cyclohexyl-1-(4'-(trifluoromethyl)-[1,1'-biphenyl]-3-yl)ethoxy)-N-((S)-2-hydroxy-3-((2-hydroxyethyl)amino)-3-oxopropyl)benzamide; or
**[14]** 4-((S)-2-cyclohexyl-1-(4'-(trifluoromethyl)-[1,1'-biphenyl]-3-yl)ethoxy)-N-((S)-2-hydroxy-3-(hydroxyamino)-3-oxopropyl)benzamide;
or a pharmaceutical acceptable salt, stereoisomer, co-crystal, prodrug or solvate thereof.

In another aspect, the invention refers to the processes for obtaining the compounds of general formula (I). Several procedures have been developed for obtaining all the compounds of the invention, and the procedures will be explained below in methods A and B.

The obtained reaction products may, if desired, be purified by conventional methods, such as crystallization and chromatography. Where the processes described below for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

### METHOD A

Method A represents a first process for synthesizing compounds according to general formula (I): 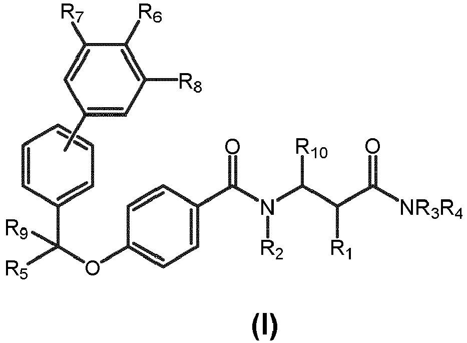
which comprises reacting a compound of formula **(XII):** 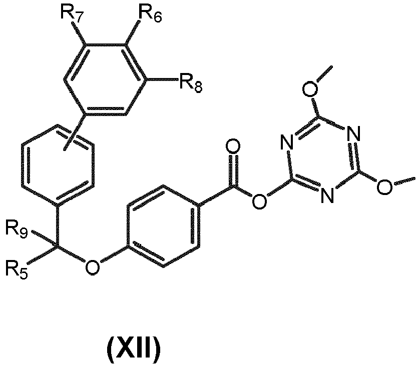
with a compound of formula **(XIII):** 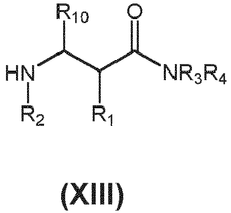
wherein **R₁, R₂, R₃, R₄, R₅, R₆, R₇, R₈, R₉** and **R₁₀** are as defined are as defined along the detailed description and the claims.

### METHOD B

Method B represents a second process for synthesizing compounds according to general formula (I). Method B allows the preparation of compounds of general formula (I) where R₃ is different from hydrogen.

Therefore, a process is described for the preparation of a compound of general formula (I): 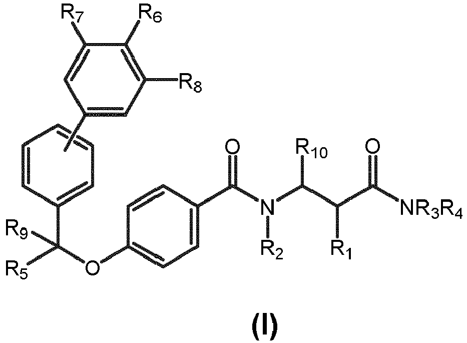
comprising the reaction between a compound of general formula (XVIII): 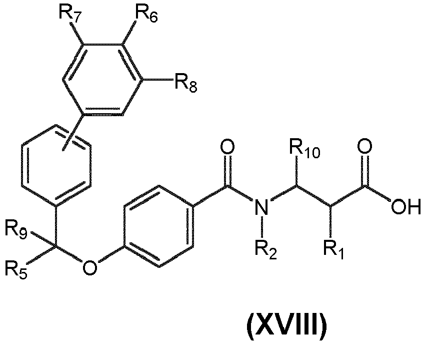
with a compound of formula (XIX): 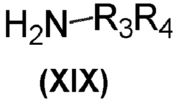
wherein **R₁, R₂, R₃, R₄, R₅, R₆, R₇, R₈, R₉** and **R₁₀** are as defined along the detailed description and the claims.

Scheme 1 below summarizes the synthetic routes of methods A and scheme 2 shows the synthetic route of method B.

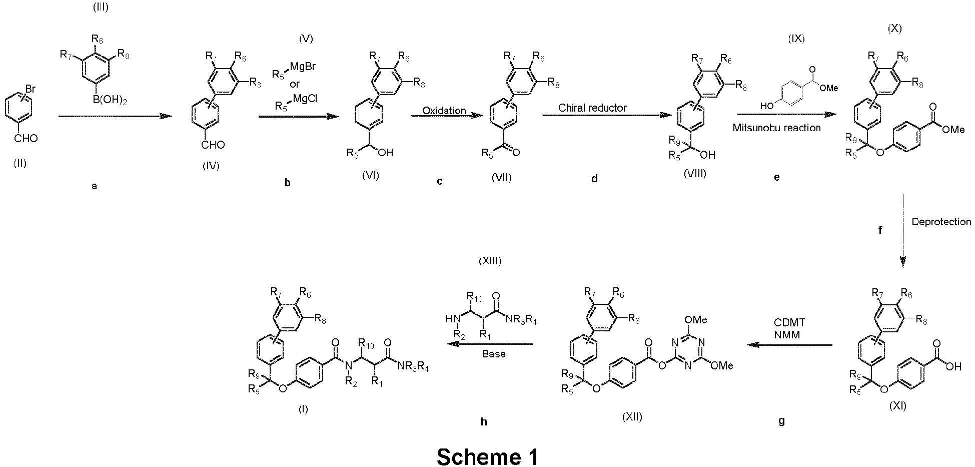

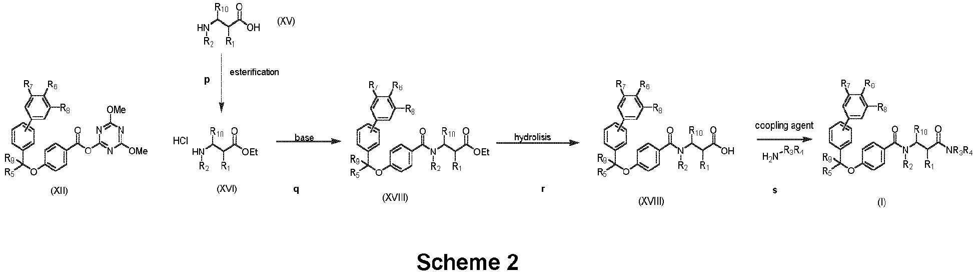

The reactions taken place in the different steps of scheme 1 (steps a to h) and scheme 2 (steps p to s) are representatively explained by means of particular embodiments in the examples.

The compounds of formula (II), (III), (V), (IX), (XIII), (XV) and (XVI) used in the methods disclosed above are commercially available or can be synthesized following common procedures described in the literature and exemplified in the synthesis of some intermediates.

Moreover, certain compounds of the present invention can also be obtained starting from other compounds of general formula (I) by appropriate conversion reactions of functional groups, in one or several steps, using well-known reactions in organic chemistry under standard experimental conditions.

In addition, a compound of general formula (I) that shows chirality can also be obtained by resolution of a racemic compound of general formula (I) either by chiral preparative HPLC or by crystallization of a diastereomeric salt or co-crystal. Alternatively, the resolution step can be carried out at a previous stage, using any suitable intermediate.

Turning to another aspect, the invention also relates to the therapeutic use of the compounds of general formula (I). As mentioned above, compounds of general formula (I) show a strong bactericidal activity against *Streptococcus sp.,* especially to *Streptococcus pneumoniae, Streptococcus pyogenes and Streptococcus agalactiae.*

Therefore, compounds of general formula (I) are useful as medicaments. And more particularly compounds of formula (I) are useful as antibiotic.

The compounds of formula (I) according to the invention are suitable for the treatment and/or prophylaxis of *Streptococcus sp.* infections.

The compounds of general formula (I) are especially suited for the treatment of infections caused by *Streptococcus pneumoniae, Streptococcus pyogenes and Streptococcus agalactiae.*

A related aspect of the invention refers to the use of compounds of general formula (I) for the manufacture of a medicament for the treatment and/or prophylaxis of *Streptococcus sp.* infections, more preferably infections caused by *Streptococcus pneumoniae, Streptococcus pyogenes and Streptococcus agalactiae.*

Another related aspect of the invention refers to a method for the treatment and/or prophylaxis of *Streptococcus sp.* infections, more preferably infections caused by *Streptococcus pneumoniae, Streptococcus pyogenes and Streptococcus agalactiae* comprising the administration of a therapeutically effective amount of a compound of general formula (I) to a subject in need thereof.

Another aspect of the invention is a pharmaceutical composition, which comprises at least a compound of general formula (I) or a pharmaceutically acceptable salt, isomer, co-crystal, prodrug or solvate thereof, and at least a pharmaceutically acceptable carrier, additive, adjuvant or vehicle.

The pharmaceutical composition of the invention can be formulated as a medicament in different pharmaceutical forms comprising at least a compound binding to the sigma receptor and optionally at least one further active substance and/or optionally at least one auxiliary substance.

The auxiliary substances or additives can be selected among carriers, excipients, support materials, lubricants, fillers, solvents, diluents, colorants, flavour conditioners such as sugars, antioxidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical composition in accordance with the invention can be adapted to any form of administration, be it orally or parenterally, for example pulmonarily, nasally, rectally and/or intravenously.

Preferably, the composition is suitable for oral or parenteral administration, more preferably for oral, intravenous, intraperitoneal, intramuscular, subcutaneous, intrathecal, rectal, transdermal, transmucosal or nasal administration.

The composition of the invention can be formulated for oral administration in any form preferably selected from the group consisting of tablets, draggers, capsules, pills, chewing gums, powders, drops, gels, juices, syrups, solutions and suspensions. The composition of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art.

Suitable preparations for parenteral applications are solutions, suspensions, reconstitutable dry preparations or sprays.

The compounds of the invention can be formulated as deposits in dissolved form or in patches, for percutaneous application.

Skin applications include ointments, gels, creams, lotions, suspensions or emulsions.

The preferred form of rectal application is by means of suppositories.

In a preferred embodiment, the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tableting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tableting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

The daily dosage for humans and animals may vary depending on factors that have their basis in the respective species or other factors, such as age, sex, weight or degree of illness and so forth. The daily dosage for humans may preferably be in the range from 1 to 2000, preferably 1 to 1500, more preferably 1 to 1000 milligrams of active substance to be administered during one or several intakes per day.

The following examples are merely illustrative of certain embodiments of the invention and cannot be considered as restricting it in any way.

### EXAMPLES

In the next examples the preparation of both intermediate compounds as well as compounds according to the invention are disclosed.

The following abbreviations are used:
anh: anhydrous
aq: aqueous
ADDP: 1,1'-(azodicarbonyl)dipiperidine
Boc₂O: di-tert-butyl dicarbonate
br s: broad singlet
C: Celsius
Cat: catalytic
CDMT: 2-chloro-4,6-dimethoxy-1,3,5-triazine
Cl-Cbz: benzyl chloroformate
DCM: dichloromethane
DIPEA: N-ethyl-N,N-diisopropylamine
DIP-CI: DIP-chloride (B-chlorodiisopinocampheylborane)
DMF: *N*,*N*-dimethylformamide
ee: enantiomeric excess
EDC·HCI: *N*-ethyl-*N'*-(3-dimethylaminopropyl)carbodiimide hydrochloride
Eq: equivalents
ESI: electrospray ionization
Et₃N: Trietilamina
Et₂O: diethyl ether
EtOAc: ethyl acetate
Ex: example
g: gram
h: hour/s
HOBt: 1-hydroxybenzotriazole
HPLC: high-performance liquid chromatography
Hz: hertz
Int: intermediate
IPA: isopropyl alcohol
L: liter
m: meter, mili, multiplet
M: molar, molecular mass
Mg: magnesium
m/z: mass-to-charge ratio
Me: methyl
MeOH: methanol
MnO₂: Manganese dioxide
MS: mass spectrometry
min: minutes
NMM: 4-methylmorpholine (N-methylmorpholine)
NMR: nuclear magnetic resonance
on: overnight
pH: -logarithm of hydrogen ion concentration
PPh₃: triphenylphosphine
Ret: retention
r.t.: room temperature
sat: saturated
TEA: triethylamine
THF: tetrahydrofuran
UPLC: ultra-performance liquid chromatography

The following methods were used to determine the HPLC-MS spectra:
**A:** Column Kinetex C18 2.6 µm, 2.1x50 mm; temperature: 40 °C; flow rate: 0.50 mL/min; A: 50 mM ammonium formate buffer pH 4 with HCOOH, B: water; C: acetonitrile, gradient A:B:C: 0.3 min in 5:85:10 + from 5:85:10 to 5:10:85 in 1.7 min + 3 min in 5:10:85.
**B:** Column Kinetex C18 2.6 µm, 2.1x50 mm; temperature: 40 °C; flow rate: 0.50 mL/min; A: 50 mM ammonium formate buffer pH 4 with HCOOH, B: water; C: acetonitrile, gradient A:B:C: 0.3 min in 5:25:70 + from 5:25:70 to 5:0:95 in 1.7 min + 3 min in 5:0:95.

The following methods were used to determine the UPLC-MS spectra:
**C:** Column Acquity CSH Fluoro-Phenyl, 1.7 µm, 2.1 x 100 mm; temperature: 35 °C; flow rate: 0.50 mL/min; A: water, B: acetonitrile; C: 50 mM ammonium acetate solution pH 6.8, gradient A:B:C: 0.5 min in 85:10:5 + from 85:10:5 to 10:85:5 in 4.5 min + 4 min in 10:85:5.
**D:** Column Acquity BEH C18 1.7 µm 2.1 x 50 mm, ; temperature: 35 °C; flow rate: 0.50 mL/min; A: 50 mM ammonium formate buffer, pH 4 with HCOOH, B: water; C: acetonitrile, gradient A:B:C: 0.5 min in 5:85:10 + from 5:85:10 to 5:10:85 in 4.5 min + 4 min in 5:10:85.
**E:** Column Acquity CSH Fluoro-Phenyl, 1.7 µm, 2.1 x 100 mm; temperature: 35 °C; flow rate: 0.50 mL/min; A: 50 mM ammonium formate buffer, pH 4 with HCOOH, B: water; C: acetonitrile, gradient A:B:C: 0.5 min in 5:85:10 + from 5:85:10 to 5:10:85 in 4.5 min + 4 min in 5:10:85.
**F:** Column Acquity CSH Phenyl-Hexyl 1.7 µm, 2.1 x 100 mm; temperature: 35 °C; flow rate: 0.50 mL/min; A: water, B: acetonitrile; C: 50 mM ammonium acetate solution pH 6.8, gradient A:B:C: 0.5 min in 85:10:5 + from 85:10:5 to 10:85:5 in 4.5 min + 4 min in 10:85:5.
**G:** Column ZORBAX Extend-C18 RRHD 1.8 µm 2.1 x 50 mm; temperature 35 °C; flow rate: 0.61 mL/min; A: Ammonium bicarbonate 10 mM, B: acetonitrile, gradient A:B 0.3 min in 98:2 + from 98:2 to 0:100 in 2.65 min + 2.05 min in 0:100.
**H:** Column ZORBAX Extend-C18 RRHD 1.8 µm 2.1 x 50 mm; temperature 35 °C; flow rate: 0.61 mL/min; A: Ammonium bicarbonate 10 mM, B: acetonitrile, C: methanol + 0.1% formic acid, gradient A:B:C 0.3 min in 98:2:0 + from 98:2:0 to 0:95:5 in 2.7 min + 1 min in 0:95:5 + from 0:95:5 to 0:100:0 in 0.1 min + 0.9 min in 0:100:0.

The following paragraps represent by way of example the preparation of certain intermediates and compounds according to formula (I) where the steps identified with letters correspond to those synthetic steps described in scheme 1 and sheme 2 above.

### Intermediate I1. 4,6-Dimethoxy-1,3,5-triazin-2-yl (S)-4-(2-cyclohexyl-1-(4'-fluoro-[1,1'-biphenyl]-4-yl)ethoxy)benzoate.

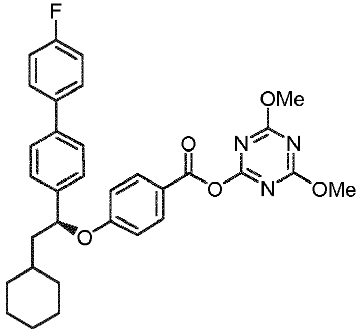

### Step b) 2-Cyclohexyl-1-(4'-fluoro-[1,1'-biphenyl]-4-yl)ethan-1-ol

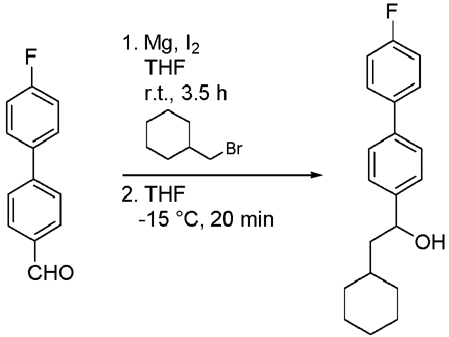

Step 1: A crystal of iodine was added to a mixture of Mg turnings (activated, 518 mg, 21.35 mmol, 1 eq) in THF (17 mL) without stirring. After 5 min, the mixture was stirred and (bromomethyl)cyclohexane (3.78 g, 3.0 mL, 21.35 mmol, 1 eq) was added in small portions (with stirring) for 2 h. It was stirred at r.t. for additional 1.5 h, rending a grey suspension, which was submitted to the next step without further purification.
Step 2: (Cyclohexylmethyl)magnesium bromide (21.35 mmol, 2.5 eq) was transferred (5 min of addition) to a -15 °C cooled solution of 4'-fluoro-[1,1'-biphenyl]-4-carbaldehyde (1.71 g, 8.54 mmol, 1,0 eq) in THF (14 mL). The mixture was stirred at -15 °C for 20 min. The reaction mixture was slowly poured into NH₄Cl (aq sat solution, 40 mL) and the aqueous layer was extracted with Et₂O (1 × 40 mL). The organic layer was washed with NH₄Cl (aq sat solution, 1x40 mL) and with water (1x30 mL), dried over anhydrous sodium sulfate, and concentrated under vacuum. Purification of the crude product by column chromatography on silica gel (EtOAc/hexanes 5-10%) yielded 2-cyclohexyl-1-(4'-fluoro-[1,1'-biphenyl]-4-yl)ethan-1-ol (1.4 g, 54% yield) as a white solid.

¹H-NMR (CDCl₃, 300 MHz) δ ppm: 7.53 (m, 4H), 7.41 (d, *J* = 7.9 Hz, 2H), 7.12 (t, *J* = 8.7 Hz, 2H), 4.84 (m, 1H), 1.86-1.39 (m, 8H), 1.32-0.90 (m, 5H).

### Step c) 2-Cyclohexyl-1-(4'-fluoro-[1,1'-biphenyl]-4-yl)ethan-1-one

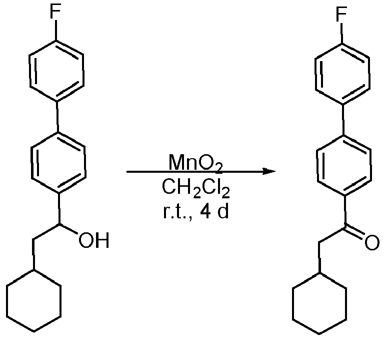

To a solution of 2-cyclohexyl-1-(4'-fluoro-[1,1'-biphenyl]-4-yl)ethan-1-ol (1.4 g, 4.691 mmol, 1 eq) in 30 mL of CH₂Cl₂ was added MnO₂ (4.63 g, 46.92 mmol, 10 eq). The solution was stirred at r.t. for 3 days. More MnO₂ (2.31 g, 23.46 mmol, 5 eq) was added, and the black suspension was stirred at r.t. ovn. The reaction mixture was filtered through a plug of celite, eluting with CH₂Cl₂ (150 mL). The filtrated was concentrated under vacuum to yield 2-cyclohexyl-1-(4'-fluoro-[1,1'-biphenyl]-4-yl)ethan-1-one (1.15 g, 82% yield) as a white solid.

¹H-NMR (CDCl₃, 300 MHz) δ ppm: 8.01 (d, *J* = 8.6 Hz, 2H), 7.67-7.54 (m, 4H), 7.16 (t, *J* = 8.6 Hz, 2H), 2.85 (d, *J* = 6.8 Hz, 2H), 1.99 (m, 1H), 1.81-1.63 (m, 5H), 1.41-0.97 (m, 5H).

### Step d) (R)-2-cyclohexyl-1-(4'-fluoro-[1,1'-biphenyl]-4-yl)ethan-1-ol

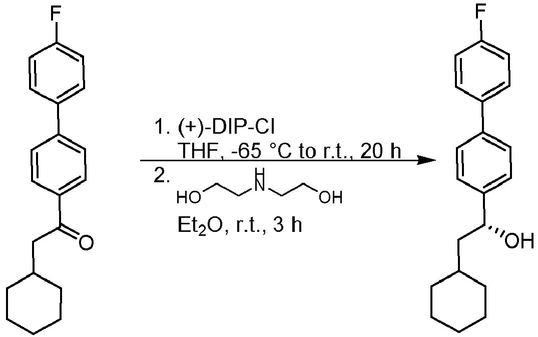

To a cooled (external T: -65 °C) solution of (+)-DIP-Cl (3.5 mL, 5.97 mmol, 1.5 eq) in 15 mL of THF was added 2-cyclohexyl-1-(4'-fluoro-[1,1'-biphenyl]-4-yl)ethan-1-one (1.18 g, 3.98 mmol, 1 eq) in THF (8 mL). The solution was allowed to reach r.t. slowly and stirred ovn. Solvent was concentrated off, the mixture was dissolved in Et₂O (20 mL) and diethanolamine (1.2 mL, 12.44 mmol, 3.12 eq) was added. The resulting white suspension was stirred at r.t. for 3 h. The mixture was diluted with hexanes (25 mL), filtered through a pad of SiO₂ eluting the product with 31% EtOAc/hexanes (400 mL). Filtrate was concentrated and the residue was flash chromatographed on SiO₂ (5-8% EtOAc/hexanes) to give (*R*)-2-cyclohexyl-1-(4'-fluoro-[1,1'-biphenyl]-4-yl)ethan-1-ol (625 mg, 52%) as a white solid.

¹H-NMR (CDCl₃, 300 MHz) δ ppm: 7.53 (m, 4H), 7.41 (d, *J* = 7.9 Hz, 2H), 7.12 (t, *J* = 8.7 Hz, 2H), 4.84 (m, 1H), 1.86-1.39 (m, 8H), 1.32-0.90 (m, 5H).

Chiral HPLC (DAICEL ChiralPak IA, IPA/hexanes): Ret=9.18 min, 98.29% (ee 96.58%).

### Step e) Methyl (S)-4-(2-cyclohexyl-1-(4'-fluoro-[1,1'-biphenyl]-4-yl)ethoxy)benzoate

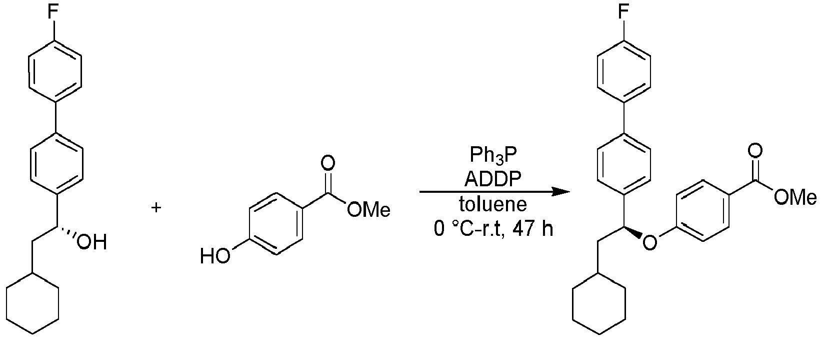

Mehyl 4-hydroxybenzoate (385 mg, 2.53 mmol, 1.3 eq) and Ph₃P (663 mg, 2.53 mmol, 1.3 eq) were added to a solution of (*R*)-2-cyclohexyl-1-(4'-fluoro-[1,1'-biphenyl]-4-yl)ethan-1-ol (581 mg, 1.95 mmol, 1 eq) in toluene (40 mL). The resulting suspension was cooled down (ice-water bath) and ADDP (638 mg, 2.53 mmol, 1.3 eq) was added. The resulting mixture was allowed to reach r.t. overnight (23 h). More mehyl 4-hydroxybenzoate (385 mg, 2.53 mmol, 1.3 eq) and Ph₃P (663 mg, 2.53 mmol, 1.3 eq) were added to the reaction. The resulting suspension was cooled down (ice-water bath) and ADDP (638 mg, 2.53 mmol, 1.3 eq) was added. The resulting mixture was allowed to slowly reach r.t. overnight (24 h). The suspension was poured into H₂O (100 mL) and extracted with EtOAc (80 mL). The organic layer was dried over anhydrous sodium sulfate, filtered and concentrated. Crude was flash chromatographed on SiO₂ (3-12% EtOAc/hexanes) to yield methyl (S)-4-(2-cyclohexyl-1-(4'-fluoro-[1,1'-biphenyl]-4-yl)ethoxy)benzoate (360 mg, 42%) as a white solid.

¹H-NMR (CDCl₃, 300 MHz) δ ppm: 7.80 (d, *J* = 9.4 Hz, 2H), 7.41 (m, 4H), 7.28 (d, *J* = 8.2 Hz, 2H), 7.01 (m, 2H), 6.78 (d, *J* = 9.4 Hz, 2H), 5.22 (dd, *J* = 9.0, 4.4 Hz, 1H), 3.75 (s, 3H), 1.91 (m, 1H), 1.78-1.50 (m, 7H), 1.22-0.81 (m, 5H).

ESI⁻-MS *m*/*z*, 431.5 (M-H).

### Step f) (S)-4-(2-Cyclohexyl-1-(4'-fluoro-[1,1'-biphenyl]-4-yl)ethoxy)benzoic acid

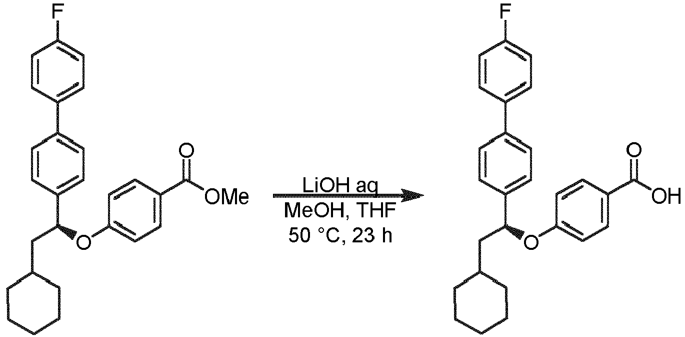

2M Lithium hydroxide aqueous solution (1.6 mL, 3.24 mmol, 4 eq) was added to a solution of methyl (S)-4-(2-cyclohexyl-1-(4'-fluoro-[1,1'-biphenyl]-4-yl)ethoxy)benzoate (350 mg, 0.81 mmol, 1 eq) in a mixture of THF (10 mL), MeOH (5 mL) and H₂O (10 mL). The resulting mixture was heated to 50 °C (external temperature) overnight (23 h). The reaction mixture was allowed to reach r.t., diluted with H₂O (15 mL), acidified with 10% HCl aq (3 mL) and extracted with CH₂Cl₂ (2x25 mL). The combined organic layers were dried over anhydrous sodium sulfate, filtered and concentrated. Crude was flash chromatographed on SiO₂ (15-40% acetone/hexanes) to give (S)-4-(2-cyclohexyl-1-(4'-fluoro-[1,1'-biphenyl]-4-yl)ethoxy)benzoic acid (335 mg, 99%) as a white solid.

¹H-NMR (DMSO-d₆, 300 MHz) δ ppm: 7.77 (d, *J* = 8.2 Hz, 2H), 7.71-7.55 (m, 4H), 7.47 (d, *J* = 8.2 Hz, 2H), 7.24 (t, *J* = 8.7 Hz, 2H), 6.99 (d, *J* = 8.7 Hz, 2H), 5.54 (dd, *J* = 8.6, 4.9 Hz, 1H), 1.99-1.36 (m, 8H), 1.25-0.94 (m, 5H).

ESI⁻-MS *m*/*z*, 417.4 (M-H).

### Step g) 4,6-Dimethoxy-1,3,5-triazin-2-yl (S)-4-(2-cyclohexyl-1-(4'-fluoro-[1,1'-biphenyl]-4-yl)ethoxy)benzoate

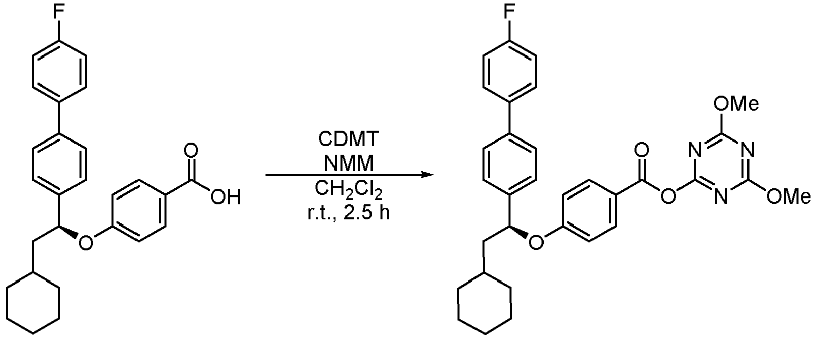

To a solution of (S)-4-(2-cyclohexyl-1-(4'-fluoro-[1,1'-biphenyl]-4-yl)ethoxy)benzoic acid (294 mg, 0.702 mmol, 1 eq) and CDMT (160 mg, 0.911 mmol, 1.3 eq) in CH₂Cl₂ (15 mL) NMM (125 µL, 1.14 mmol, 1.62 eq) was added. The solution was stirred at r.t. for 2.5 h. The crude volatiles were removed under reduced pressure. Purification of the crude product by flash column chromatography on silica (EtOAc/hexanes 12-30%) afforded 4,6-dimethoxy-1,3,5-triazin-2-yl (S)-4-(2-cyclohexyl-1-(4'-fluoro-[1,1'-biphenyl]-4-yl)ethoxy)benzoate (349 mg, 89% yield) as a pale yellow solid.

¹H-NMR (CDCl₃, 300 MHz) δ ppm 8.00 (d, *J* = 8.8 Hz, 2H), 7.52 (m, 4H), 7.37 (d, *J* = 8.2 Hz, 2H), 7.10 (t, *J* = 8.8 Hz, 2H), 6.93 (d, *J* = 8.8 Hz, 2H), 5.37 (m, 1H), 4.04 (s, 6H), 2.02-1.61 (m, 8H), 1.32-0.97 (m, 5H).

ESI⁺-MS *m*/*z,* 558.5 (M+H).

This method was used for the preparation of intermediates Int 2-Int 8 using suitable starting materials:

| **Int** | **Structure** | **Chemical name** | **MS (M+H)** | **Ret (min)** | **Method LC-MS** |
|---|---|---|---|---|---|
| Int 1 | 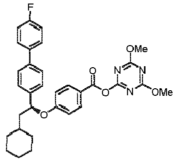 | 4,6-Dimethoxy-1,3,5-triazin-2-yl (S)-4-(2-cyclohexyl-1-(4'-fluoro-[1,1'-biphenyl]-4-yl)ethoxy)benzoate | 558.5 | 2.986 | **B** |
| Int 2 | 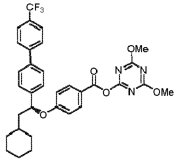 | 4,6-Dimethoxy-1,3,5-triazin-2-yl (S)-4-(2-cyclohexyl-1-(4'-(trifluoromethyl)-[1,1'-biphenyl]-4-yl)ethoxy)benzoate | 608.6 | 4.779 | **A** |
| Int 3 | 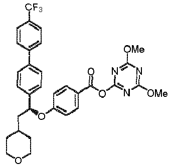 | 4,6-Dimethoxy-1,3,5-triazin-2-yl (S)-4-(2-(tetrahydro-2H-pyran-4-yl)-1-(4'-(trifluoromethyl)-[1,1'-biphenyl]-4-yl)ethoxy)benzoate | 610.1 | 3.643 | **A** |
| Int 4 | 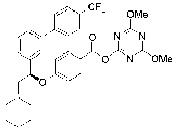 | 4,6-Dimethoxy-1,3,5-triazin-2-yl (S)-4-(2-cyclohexyl-1-(4'-(trifluoromethyl)-[1,1'-biphenyl]-3-yl)ethoxy)benzoate | 608.3 | 4.534 | **A** |
| Int 5 | 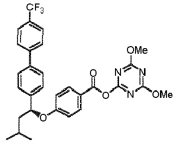 | 4,6-Dimethoxy-1,3,5-triazin-2-yl (S)-4-(3-methyl-1-(4'-(trifluoromethyl)-[1,1'-biphenyl]-4-yl)butoxy)benzoate | ¹H-NMR (CDCl₃, 300 MHz) δ ppm 8.02 (d, *J* = 8.8 Hz, 2H), 7.67 (m, 4H), 7.56 (d, *J* = 8.2 Hz, 2H), 7.42 (d, *J* = 8.2 Hz, 2H), 6.94 (d, *J* = 8.8 Hz, 2H), 5.31 (m, 1H), 4.04 (s, 6H), 2.10-1.84 (m, 2H), 1.66 (m, 1H), 0.99 (m, 6H). | | |
| Int 6 | 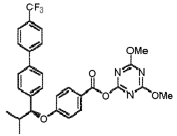 | 4,6-Dimethoxy-1,3,5-triazin-2-yl (S)-4-(2-methyl-1-(4'-(trifluoromethyl)-[1,1'-biphenyl]-4-yl)propoxy)benzoate | 554.2 | 4.019 | **A** |
| Int 7 | 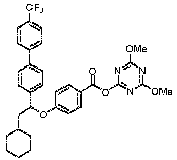 | 4,6-Dimethoxy-1,3,5-triazin-2-yl 4-(2-cyclohexyl-1-(4'-(trifluoromethyl)-[1,1'-biphenyl]-4-yl)ethoxy)benzoate | 608.6 | 4.779 | **A** |
| Int 8 | 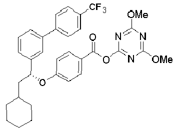 | 4,6-Dimethoxy-1,3,5-triazin-2-yl (R)-4-(2-cyclohexyl-1-(4'-(trifluoromethyl)-[1,1'-biphenyl]-3-yl)ethoxy)benzoate | - | 3.196 | **G** |

### Example 1. N-((S)-3-Amino-2-hydroxy-3-oxopropyl)-4-((S)-2-cyclohexyl-1-(4'-fluoro-[1,1'-biphenyl]-4-yl)ethoxy)benzamide

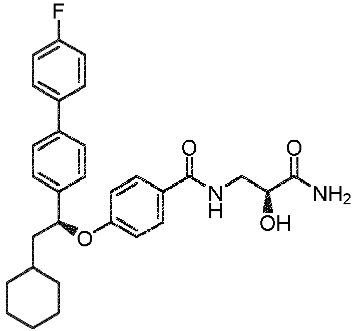

### Step i) (S)-3-(((Benzyloxy)carbonyl)amino)-2-hydroxypropanoic acid

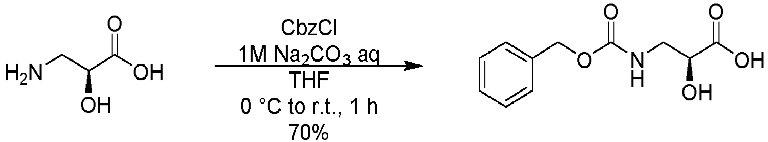

Cl-Cbz (2.4 mL, 17.02 mmol, 1.12 eq) was dropwise added to a solution of L-isoserine (1.6 g, 15.22 mmol, 1 eq) in a mixture of THF (25 mL) and 1M Na₂CO₃ aq (20 mL, 20.00 mmol, 1.31 mmol) cooled down by an ice-water bath. Cooling bath was removed and the reaction mixture was stirred for 1 h. The resulting mixture was diluted with H₂O (80 mL), washed with Et₂O (80 mL), acidified with 10% HCl aq (20 mL) and extracted with CH₂Cl₂ (2 x 50 mL). The organic layer was dried over anhydrous sodium sulfate, filtered and concentrated to give the desired product (2.55 g, 70%) as a white solid.

### Step j) (S)-3-(((Benzyloxy)carbonyl)amino)-2-hydroxypropanoic acid

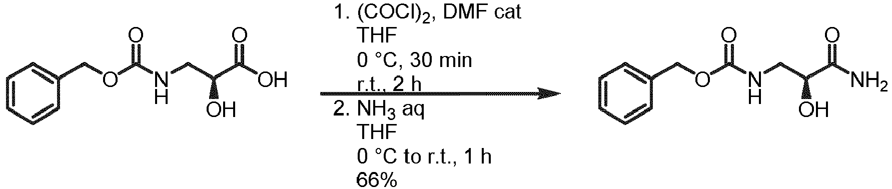

(COCl)₂ (0.35 mL, 4.09 mmol, 1.12 eq) was dropwise added to a solution of the acid (871 mg, 3.64 mmol, 1 eq) in THF (15 mL) and DMF (3 drops), cooled down by an ice-water bath (gas evolution). The resulting solution was stirred at low temperature for 0.5 h, and at r.t. for 2 h. The solution was concentrated, and the residue was dissolved in THF (10 mL) and it was added (*via* canula) to a mixture of THF (15 mL) and 25% aqueous ammonia (15 mL) cooled down by an ice-water bath. Cooling bath was removed and the resulting suspension was stirred for 1 h. The resulting mixture was diluted with H₂O (15 mL), acidified with 10% HCl aq (20 mL) and extracted with CH₂Cl₂ (3 x 50 mL) and with CH₂Cl₂/IPA (4:1, 2x 20 mL). The combined organic layers were dried over anhydrous sodium sulfate, filtered and concentrated. The solid obtained (950 mg, crude) was suspended in CH₂Cl₂ (20 mL), stirred at r.t. for 1 h, filtered and dried under high vacuum to give (S)-3-(((benzyloxy)carbonyl)amino)-2-hydroxypropanoic acid (578 mg, 66%) as a white solid.

¹H-NMR (DMSO-d₆, 300 MHz) δ ppm: 7.40-7.27 (m, 5H), 7.25-7.10 (m, 2H), 5.59 (m, 1H), 5.01 (s, 2H), 3.87 (m, 1H), 3.04 (dt, *J* = 14.1, 7.2 Hz, 1H).

ESI⁺-MS *m*/*z,* 239.1.

### Step k) (S)-3-Amino-2-hydroxypropanamide

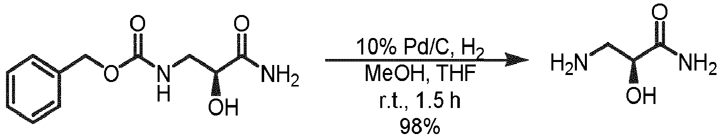

Pd/C 10% (wet) (450 mg, 0.243 mmol, 0.1 eq) was added to a solution of (S)-3-(((benzyloxy)carbonyl)amino)-2-hydroxypropanoic acid (578 mg, 2.426 mmol, 1 eq) in a mixture of MeOH (20 mL) and THF (15 mL). The resulting mixture was stirred at r.t. under H₂ atmosphere (balloon) for 1.5 h. The suspension was filtered through a short pad of celite, eluting with MeOH (150 mL) and EtOAc (30 mL). The solvent was concentrated off, to give the desired product as a colourless oil. The crude residue (264 mg) was slurred with Et₂O (4 mL), solvent was discarded, and the solid obtained was dried under high vacuum to give (S)-3-amino-2-hydroxypropanamide (250 mg, 98%) as a white solid. ¹H-NMR (DMSO-d₆, 300 MHz) δ ppm: 7.14 (d, *J =* 14.7 Hz, 2H), 3.72 (dd, *J* = 6.7, 4.1 Hz, 1H), 2.74 (dd, *J* = 13.0, 4.1 Hz, 1H), 2.58 (dd, *J* = 13.0, 6.7 Hz, 1H).

### Step h) Title compound.

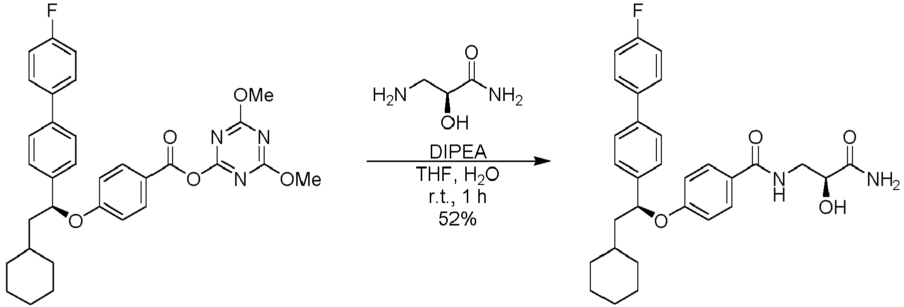

A solution of 4,6-dimethoxy-1,3,5-triazin-2-yl (S)-4-(2-cyclohexyl-1-(4'-fluoro-[1,1'-biphenyl]-4-yl)ethoxy)benzoate, Intermediate I1 ( 95 mg, 0.17 mmol, 1 eq) in THF (5 mL) was added to a solution of (S)-3-amino-2-hydroxypropanamide (100 mg, 0.96 mmol, 5.64 eq) and DIPEA (160 µL, 0.934 mmol, 5.49 eq) in a mixture of THF (10 mL) and H₂O (2 mL). The resulting solution was stirred at r.t. for 1 h, poured into H₂O (30 mL), acidified with 10% HCl aq solution (2 mL) and extracted with CH₂Cl₂ (2 x 30 mL). The organic layer was dried over anhydrous sodium sulfate, filtered and concentrated. The residue was slurred with Et₂O (6 mL), the solid was collected by filtration, washed 3 times with Et₂O (4 mL) and dried under high vacuum at 50 °C to give N-((S)-3-amino-2-hydroxy-3-oxopropyl)-4-((S)-2-cyclohexyl-1-(4'-fluoro-[1,1'-biphenyl]-4-yl)ethoxy)benzamide (45 mg, 52%) as a white solid.

¹H-NMR (DMSO-d₆, 300 MHz) δ ppm: 8.16 (t, *J* = 5.6 Hz, 1H), 7.73-7.65 (m, 4H), 7.60 (d, *J* = 8.7 Hz, 2H), 7.47 (d, *J* = 8.7 Hz, 2H), 7.29-7.14 (m, 4H), 6.98 (d, *J* = 8.7 Hz, 2H), 5.63 (d, *J* = 5.6 Hz, 1H), 5.55 (m, 1H), 3.98 (m, 1H), 3.56 (m, 1H), 3.23 (m, 1H), 1.97-1.37 (m, 8H), 1.24-0.97 (m, 5H).

ESI⁺-MS *m*/*z,* 505.5.

This method was used for the preparation of examples 2-11 using suitable starting materials:

| **Ex** | **Structure** | **Chemical name** | **MS (M+H)** | **Ret (min)** | **Method LC-MS** |
|---|---|---|---|---|---|
| 1 | 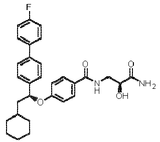 | *N*-((S)-3-Amino-2-hydroxy-3-oxopropyl)-4-((S)-2-cyclohexyl-1-(4'-fluoro-[1,1'-biphenyl]-4-yl)ethoxy)benzamide | 505.5 | 4.728 | **C** |
| 2 | 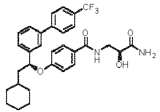 | *N*-((S)-3-Amino-2-hydroxy-3-oxopropyl)-4-((S)-2-cyclohexyl-1-(4'-(trifluoromethyl)-[1,1'-biphenyl]-3-yl)ethoxy)benzamide | 555.6 | 4.890 | **E** |
| 3 | 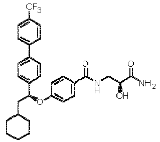 | *N*-((S)-3-Amino-2-hydroxy-3-oxopropyl)-4-((S)-2-cyclohexyl-1-(4'-(trifluoromethyl)-[1,1'-biphenyl]-4-yl)ethoxy)benzamide | 555.5 | 4.913 | **C** |
| 4 | 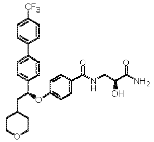 | *N*-((S)-3-amino-2-hydroxy-3-oxopropyl)-4-((S)-2-(tetrahydro-2H-pyran-4-yl)-1-(4'-(trifluoromethyl)-[1,1'-biphenyl]-4-yl)ethoxy)benzamide | 557.5 | 4.454 | **E** |
| 5 | 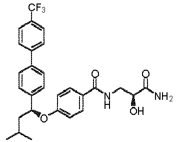 | *N*-((S)-3-amino-2-hydroxy-3-oxopropyl)-4-((S)-3-methyl-1-(4'-(trifluoromethyl)-[1,1'-bipheny]-4-yl)butoxy)benzamide | 515.4 | 4.726 | **F** |
| 6 | 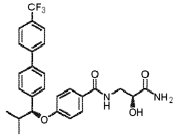 | *N*-((S)-3-amino-2-hydroxy-3-oxopropyl)-4-((S)-2-methyl-1-(4'-(trifluoromethyl)-[1,1'-biphenyl]-4-yl)propoxy)benzamide | 501.4 | 4.631 | **C** |
| 7 | 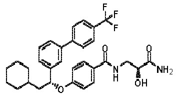 | N-((S)-3-amino-2-hydroxy-3-oxopropyl)-4-((R)-2-cyclohexyl-1-(4'-(trifluoromethyl)-[1,1'-biphenyl]-3-yl)ethoxy)benzamide | 553.4 (M-H) | 2.81 | **H** |
| 8 | 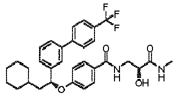 | 4-((S)-2-cyclohexyl-1-(4'-(trifluoromethyl)-[1,1'-biphenyl]-3-yl)ethoxy)-N-((S)-2-hydroxy-3-(methylamino)-3-oxopropyl)benzamide | 569.4 | 2.771 | **G** |
| 9 | 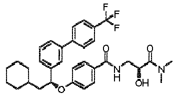 | 4-((S)-2-cyclohexyl-1-(4'-(trifluoromethyl)-[1,1'-biphenyl]-3-yl)ethoxy)-N-((S)-3-(dimethylamino)-2-hydroxy-3-oxopropyl)benzamide | | | |
| 10 | 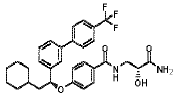 | N-((R)-3-amino-2-hydroxy-3-oxopropyl)-4-((S)-2-cyclohexyl-1-(4'-(trifluoromethyl)-[1,1'-biphenyl]-3-yl)ethoxy)benzamide | | | |
| 11 | 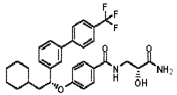 | N-((R)-3-amino-2-hydroxy-3-oxopropyl)-4-((R)-2-cyclohexyl-1-(4'-(trifluoromethyl)-[1,1'-biphenyl]-3-yl)ethoxy)benzamide | | | |

### Example 12. N-((R)-4-amino-4-oxobutan-2-yl)-4-((S)-2-cyclohexyl-1-(4'-(trifluoromethyl)-[1,1'-biphenyl]-4-yl)ethoxy)benzamide

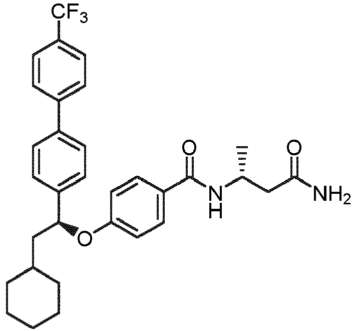

### Step I) (R)-3-((tert-Butoxycarbonyl)amino)butanoic acid

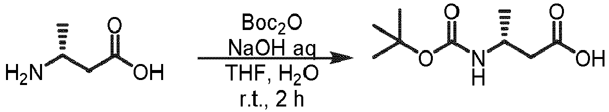

Boc₂O (2.1 g, 9.62 mmol, 1.14 eq) was added to a solution of (R)-homo-beta-alanine (867 mg, 8.41 mmol, 1 eq) in a mixture of THF (25 mL), H₂O (10 mL) and 2.5 M NaOH aq solution (5 mL). The resulting mixture was stirred at r.t. for 2 h. The mixture was diluted with H₂O (30 mL), washed with Et₂O (40 mL), acidified with 10% HCl aq solution (8 mL) and extracted with CH₂Cl₂ (2 x 40 mL). The combined organic layers were dried over anhydrous sodium sulfate, filtered and concentrated to give (R)-3-((*tert-*butoxycarbonyl)amino)butanoic acid (1.17 g, 68%) as a colourless oil.

### Step m) tert-butyl (R)-(4-Amino-4-oxobutan-2-yl)carbamate

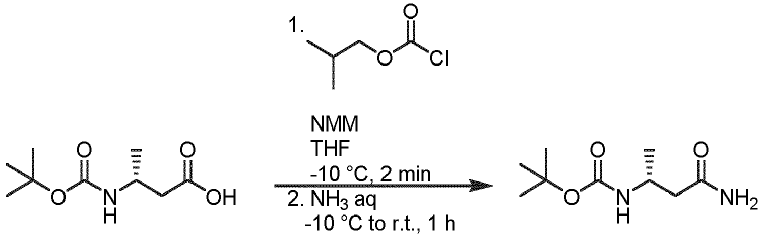

NMM (0.65 mL, 5.92 mmol, 1.03 eq) and isobutylchloroformate (0.76 mL, 5.87 mmol, 1.02 eq) were added to a solution of (R)-3-((*tert*-butoxycarbonyl)amino)butanoic acid (1.17 g, 5.76 mmol, 1 eq) in THF (25 mL) cooled down by an ice-EtOH bath (external temperature: -10 °C). The resulting mixture was stirred at low temperature for 2 min and ammonia (3 mL, 25% solution in H₂O) was added. Cooling bath was removed, the reaction mixture was stirred for 1 h and it was diluted with EtOAc (60 mL), washed with H₂O (2 x 60 mL) and with 0.8 M HCl aq solution (25 mL), dried over anhydrous sodium sulfate, filtered and concentrated to yield tert-butyl (R)-(4-amino-4-oxobutan-2-yl)carbamate (696 mg, 59%) as a white solid.

¹H-NMR (CDCl₃, 300 MHz) δ ppm: 5.99 (br s, 1H), 5.48 (br s, 1H), 5.06 (br s, 1H), 3.98 (m, 1H), 2.43 (d, *J* = 5.9 Hz, 2H), 1.43 (s, 9H), 1.25 (d, *J* = 6.7 Hz, 3H).

### Step n) (R)-3-Aminobutanamide hydrochloride hydrochloride

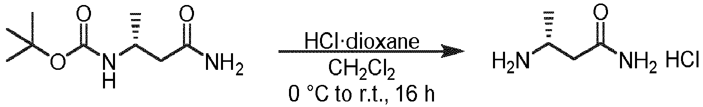

HCl·dioxane (5.1 mL, 20.47 mmol, 6 eq) was added to a suspension of *tert*-butyl (R)-(4-amino-4-oxobutan-2-yl)carbamate (690 mg, 3.41 mmol, 1 eq) in CH₂Cl₂ (25 mL) cooled down by an ice-water bath. The resulting mixture was allowed to reach r.t. overnight (16 h). The resulting suspension was filtered, and the solid was washed with CH₂Cl₂ (10 mL) and with Et₂O (15 mL) and dried under high vacuum to yield (R)-3-aminobutanamide hydrochloride (441 mg, 93%) as a hygroscopic white solid.

¹H-NMR (DMSO-d₆, 300 MHz) δ ppm: 8.12 (br s, 3H), 7.64 (br s, 1H), 7.05 (br s, 1H), 3.41 (m, 1H), 2.35 (m, 2H), 1.17 (d, *J* = 6.5 Hz, 3H).

### Step o) Title compound

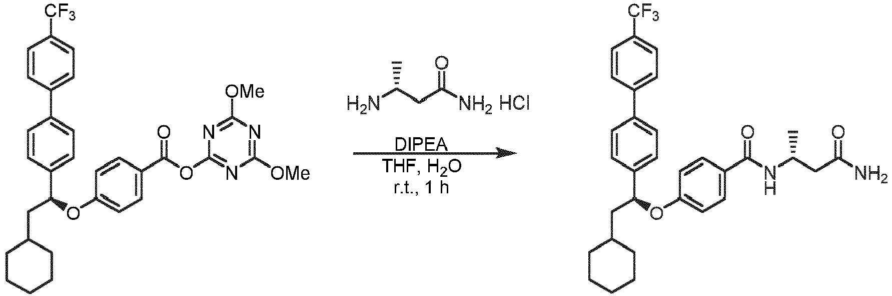

4,6-Dimethoxy-1,3,5-triazin-2-yl (S)-4-(2-cyclohexyl-1-(4'-(trifluoromethyl)-[1,1'-biphenyl]-4-yl)ethoxy)benzoate (intermediate I2, 90 mg, 0.148 mmol, 1 eq) was added to a solution of (R)-2-aminopropanamide hydrochloride (75 mg, 0.602 mmol, 4.06 eq) and DIPEA (0.25 mL, 1.46 mmol, 9.86 eq) in a mixture of THF (12 mL) and H₂O (2 mL). The reaction mixture was stirred at r.t. for 1 h and it was diluted with CH₂Cl₂ (40 mL), washed with HCl (5% aqueous solution, 1x20 mL), dried over anhydrous sodium sulfate, filtered and concentrated. Crude was flash chromatographed on SiO₂ (25-50% acetone/hexanes) to give *N*-((R)-1-amino-1-oxopropan-2-yl)-4-((S)-2-cyclohexyl-1-(4'-(trifluoromethyl)-[1,1'-biphenyl]-4-yl)ethoxy)benzamide (68 mg, 85%) as a white solid. ¹H-NMR (DMSO-d₆, 300 MHz) δ ppm: 8.13 (d, *J* = 7.5 Hz, 1H), 7.87 (d, *J* = 8.3 Hz, 2H), 7.83-7.66 (m, 6H), 7.54 (d, *J* = 8.3 Hz, 2H), 7.26 (brs, 1H), 7.00 (d, *J* = 8.7 Hz, 2H), 6.90 (brs, 1H), 5.58 (m, 1H), 4.35 (m, 1H), 2.01-1.72 (m, 4H), 1.71-1.35 (m, 4H), 1.27 (d, *J* = 7.5 Hz, 3H), 1.24-0.90 (m, 7H)

HPLC-MS (Method B): Rt: 5.663 min; ESI+ MS: m/z 553.5

### Example 13. 4-((S)-2-Cyclohexyl-1-(4'-(trifluoromethyl)-[1,1'-biphenyl]-3-yl)ethoxy)-N-((S)-2-hydroxy-3-((2-hydroxyethyl)amino)-3-oxopropyl)benzamide

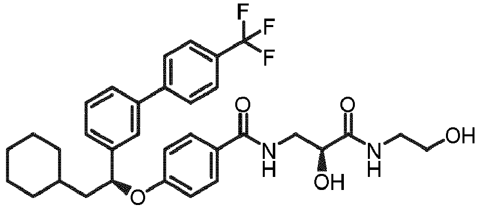

### Step p) Ethyl (S)-3-amino-2-hydroxypropanoate hydrochloride.

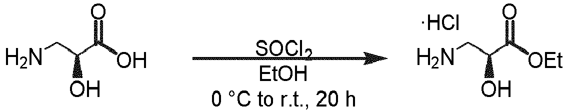

EtOH (20 mL) was cooled down to 0°C (ice-water bath) and SOCl₂ (1.6 mL, 22.055 mmol, 1.84 eq) was added. After 5 min, L-isoserine (1.26 g, 11.989 mmol, 1 eq) was added. The suspension was allowed to reach r.t. slowly on (20 h). The crude volatiles were removed under reduced pressure to yield ethyl (S)-3-amino-2-hydroxypropanoate hydrochloride (2.2 g, yield >theoretical) as a yellow oil, which was submitted to next step without further purification.

¹H-NMR (DMSO-d6, 300 MHz) δ ppm: 8.15 (s, 3H), 6.31 (d, J = 5.5 Hz, 1H), 4.34 (dd, J = 9.2, 4.8 Hz, 1H), 4.14 (c, J = 7.0 Hz, 2H), 3.09 (m, 1H), 2.89 (m, 1H), 1.22 (t, J = 7.0 Hz, 3H).

### Step q) Ethyl (S)-3-(4-((S)-2-cyclohexyl-1-(4'-(trifluoromethyl)-[1,1'-biphenyl]-3-yl)ethoxy)benzamido)-2-hydroxypropanoate.

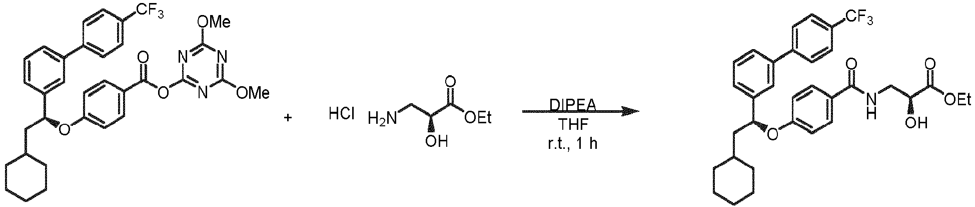

A solution of 4,6-dimethoxy-1,3,5-triazin-2-yl (S)-4-(2-cyclohexyl-1-(4'-(trifluoromethyl)-[1,1'-biphenyl]-3-yl)ethoxy)benzoate, Intermediate 4, (244 mg, 0.401 mmol, 1 eq) in THF (5 mL) was added to a solution of DIPEA (275 µL,1.606 mmol, 4.01 eq) and the aminoester (204 mg, 1.202 mmol, 3 eq) in a mixture of THF (10 mL) and H₂O (2 mL). The resulting mixture was stirred at r.t. for 1 h, diluted with EtOAc (20 mL), washed with 5% HCl aq (15 mL), dried over anhydrous sodium sulfate, filtered and concentrated. Purification of the crude product by column chromatography on silica gel (19-40% EtOAc/hexanes) gave ethyl (S)-3-(4-((S)-2-cyclohexyl-1-(4'-(trifluoromethyl)-[1,1'-biphenyl]-3-yl)ethoxy)benzamido)-2-hydroxypropanoate (218 mg, 93%) as a white solid.

¹H-NMR (CDCl₃, 300 MHz) δ ppm: 7.71-7.57 (m, 6H), 7.54-7.31 (m, 4H), 6.87 (d, J = 8.8 Hz, 2H), 6.36 (m, 1H), 5.31 (m, 1H), 4.32 (m, 1H), 4.22 (c, J = 7.0 Hz, 2H), 3.76 (m, 2H), 2.02-1.53 (m, 12H), 1.27 (t, J = 7.0 Hz, 3H).

ESI⁺-MS m/z, 584.6 (M+H).

### Step r) (S)-3-(4-((S)-2-Cyclohexyl-1-(4'-(trifluoromethyl)-[1,1'-biphenyl]-3-yl)ethoxy)benzamido)-2-hydroxypropanoic acid.

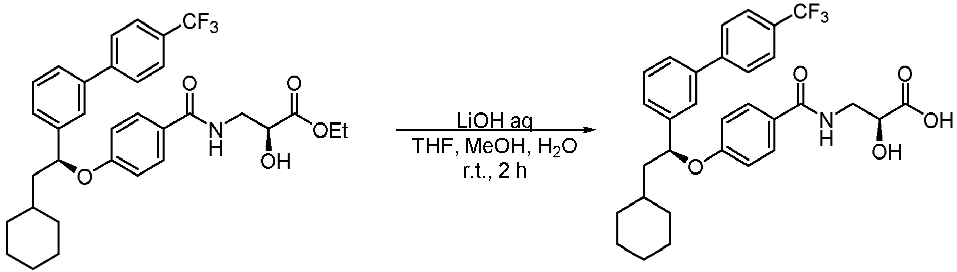

Lithium hydroxide solution (2M, 720 µL, 1.44 mmol, 4 eq) was added to a solution of ethyl (S)-3-(4-((S)-2-cyclohexyl-1-(4'-(trifluoromethyl)-[1,1'-biphenyl]-3-yl)ethoxy)benzamido)-2-hydroxypropanoate (210 mg, 0.359 mmol, 1 eq) in a mixture of THF (6 mL), H₂O (6 mL) and MeOH (3 mL). The resulting solution was stirred at r.t. for 2 h and it was diluted with H₂O (15 mL), acidified with 10% HCl aq (2 mL) and extracted with CH₂Cl₂ (2x15 mL). The combined organic layers were dried over anhydrous sodium sulfate, filtered and concentrated under vacuum, to afford 206 mg of a white solid (crude). This solid was slurred with hexanes (4 mL). The solid was collected by filtration, rinsed 3 times with 4 mL of hexanes and dried under high vacuum to give title compound as a white foam (155 mg). The foam was suspended in a mixture CH₂Cl₂/ pentane (1:1, 4 mL) and stirred at r.t. for 10 min. The solid was collected by filtration, rinsed 3 times with 4 mL of pentane and dried under high vacuum to give (S)-3-(4-((S)-2-cyclohexyl-1-(4'-(trifluoromethyl)-[1,1'-biphenyl]-3-yl)ethoxy)benzamido)-2-hydroxypropanoic acid (137 mg, 68%) as a white solid.

¹H-NMR (DMSO-d₆, 300 MHz) δ ppm: 8.22 (t, J = 5.8 Hz, 1H), 7.88-7.76 (m, 5H), 7.70 (d, J = 8.6 Hz, 2H), 7.62 (m, 1H), 7.47 (d, J = 4.6 Hz, 2H), 7.01 (d, J = 8.6 Hz, 2H), 5.60 (dd, J = 8.8, 4.6 Hz, 1H), 4.11 (dd, J = 7.2, 4.8 Hz, 1H), 3.51 (m, 2H), 2.03-139 (m, 8H), 1.23-0.90 (m, 5H).

ESI⁺-MS m/z, 556.5 (M+H).

### Step s) 4-((S)-2-Cyclohexyl-1-(4'-(trifluoromethyl)-[1,1'-biphenyl]-3-yl)ethoxy)-N-((S)-2-hydroxy-3-((2-hydroxyethyl)amino)-3-oxopropyl)benzamide.

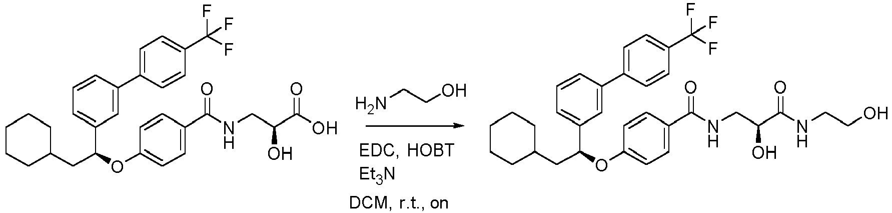

To a solution of (S)-4-(2-cyclohexyl-1-(4'-(trifluoromethyl)-[1,1'-biphenyl]-3-yl)ethoxy)benzoic acid (98 mg, 0.176 mmol, 1 eq), EDC.HCl (55 mg, 0.352 mmol, 2 eq), HOBt (54 mg, 0.535 mmol, 2eq) and TEA (99 µL,, 0.706 mmol, 45eq) in CH₂Cl₂ (8 mL), 2-aminoethan-1-ol (32 mg, 0.529 mmol, 3 eq) was added. The solution was stirred at r.t. overnight. The resulting solution was diluted with water (10 mL) and extracted with CH₂Cl₂ (2x15 mL). The combined organic layers were dried over anhydrous sodium sulfate, filtered and concentrated under vacuum. Purification of the crude product by flash gold chromatography on silica gel (EtOAc/hexanes) yielded 4-((S)-2-cyclohexyl-1-(4'-(trifluoromethyl)-[1,1'-biphenyl]-3-yl)ethoxy)-N-((S)-2-hydroxy-3-((2-hydroxyethyl)amino)-3-oxopropyl) benzamide (21 mg, 20%) as a white solid.

¹H NMR (DMSO-d₆, 400 MHz) δ ppm: 8.21 (t, *J* = 5.7 Hz, 1H), 7.93 - 7.77 (m, 5H), 7.75 - 7.67 (m, 3H), 7.61 (td, *J* = 4.5, 1.9 Hz, 1H), 7.46 (d, *J* = 5.2 Hz, 2H), 7.01 (d, *J* = 9.0 Hz, 2H), 5.80 (d, *J* = 5.4 Hz, 1H), 5.61 (dd, *J* = 8.8, 4.7 Hz, 1H), 4.68 (t, *J* = 5.5 Hz, 1H), 4.09 - 3.96 (m, 1H), 3.56 (dt, *J* = 13.4, 4.8 Hz, 1H), 3.38 (q, *J* = 6.0 Hz, 2H), 3.26 - 3.07 (m, 3H), 2.01 - 1.90 (m, 1H), 1.86 - 1.74 (m, 2H), 1.72 - 1.55 (m, 4H), 1.54 - 1.41 (m, 1H), 1.29 - 1.08 (m, 3H), 1.08 - 0.93 (m, 2H).

ESI⁺-MS m/z, 599.4 (M+H).

This method was used for the preparation of example 14 using suitable starting materials:

| **Ex** | **Structure** | **Chemical name** | **MS (M+H)** | **Ret (min)** | **Method LC-MS** |
|---|---|---|---|---|---|
| 13 | 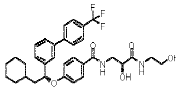 | 4-((S)-2-cyclohexyl-1-(4'-(trifluoromethyl)-[1,1'-biphenyl]-3-yl)ethoxy)-N-((S)-2-hydroxy-3-((2-hydroxyethyl)amino)-3-oxopropyl)benzamide | 599.4 | 2.840 | **G** |
| 14 | 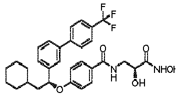 | 4-((S)-2-cyclohexyl-1-(4'-(trifluoromethyl)-[1,1'-biphenyl]-3-yl)ethoxy)-N-((S)-2-hydroxy-3-(hydroxyamino)-3-oxopropyl)benzamide | | | |

### Examples of biological activity

### Experimental procedure: MIC Test. Microdilution method

To evaluate the antimicrobial activity of the compounds, ATCC strains of *Streptococcus pneumoniae* and *Streptococcus pyogenes* were used. The minimal inhibitory concentrations (MICs) of the compounds were determined by microdilution assays, according to CLSI methodology.

### Microplate Set-up: Preparation of compounds

3mg of compound were weighted in a 4mL glass vials and were dissolved in properly volume of DMSO 100% (Reference: D2438-50, Sigma) to reach 12,8 mg/mL. (Cmax.).

### "Mother" plate

In 96-well plates U-form (Reference: 650161, Greiner Bio-One):
- Dispense 50µL of the assayed compounds prepared in DMSO 100% at 12,8 mg/mL in each well in column 1. (8 different compounds per plate, row 1 to row 8)
- Dispense 25µL of DMSO 100% in all wells in columns 2 to 12.
- With a multichannel pipette, aspire 25µL of column 1 and dispense in column 2. Mix the contents in column 2 several times and aspire 25uL. Dispense in column 3.
- Repeat the process until column 10. In column 10, aspire 25µL of the content and discard.
- The final mother plate map is all values in mg/mL)

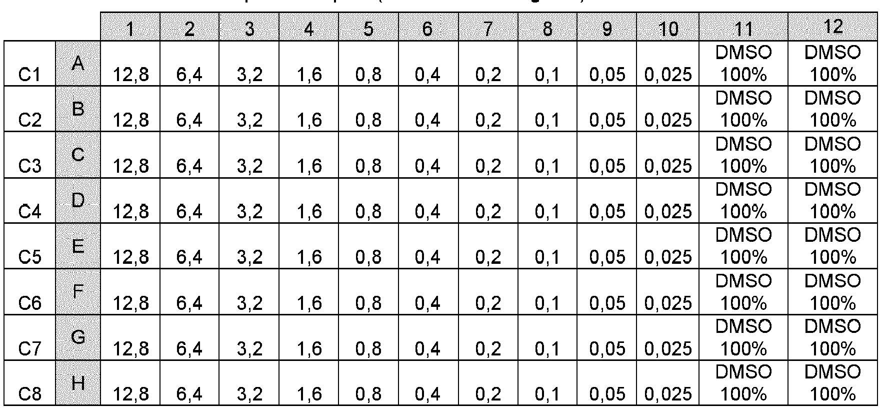

### "Daughter" plates:

In 96-well plates U-form:
- With a multichannel pipette (in dispensing mode), aspire a properly volume (consider the number of copies of the mother plate do you want to perform) of all wells in mother plate, to dispense **1µL/well** in daughter plates.

### NOTE:

Tecan instrument (FREEDOM EVO 100) was used to make the mother and daughter plates.

### MIC test: inoculation of bacteria

Bacterial suspension will be prepared and normalized to 0,5 McFarland turbidity standard (approximately 10⁸ CFU/mL) using a physiological saline solution (sodium chloride 0,9%). Then, prepared a 1/1000 dilution (approximately 10⁵ CFU/mL) of the suspension in appropriate media (CLSI Standard) and inoculate 99µL in each well in daughter plates (only columns 1 to 11) using a multidrop^{™} Combi Reagent Dispenser. In column 12, only 99µL of media will be dispense as a sterility control.

The final plate map is (all values in µg/mL):

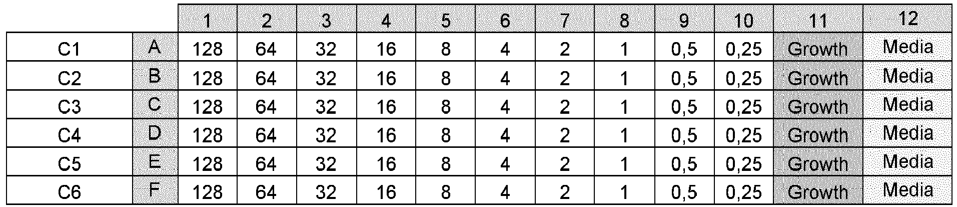

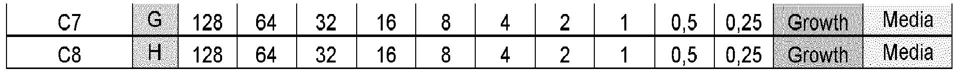

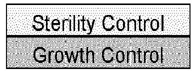

Plates will be incubated 18-20 h at 37°C. MIC values will be considered as the concentration of compounds at which the growth will be inhibited => ≥80%, compared to growth control.

### Examples of biological activity

| **Ex** | **Activity (µg/mL)** |
|---|---|
| 1 | Spn 4 Spy 4 |
| 2 | Spn 2 |
| | Spy 1 |
| 4 | Spn >128 |
| | Spy >128 |
| 5 | Spn >128 |
| | Spy >128 |
| 6 | Spn 4 |
| | Spy 4 |
| 8 | Spn 4 |
| | Spy 4 |

| | |
|---|---|
| Spn: Streptococcus pneumoniae Spy: Streptococcus pyogenes | |

## Claims

1. A compound of general formula (I): 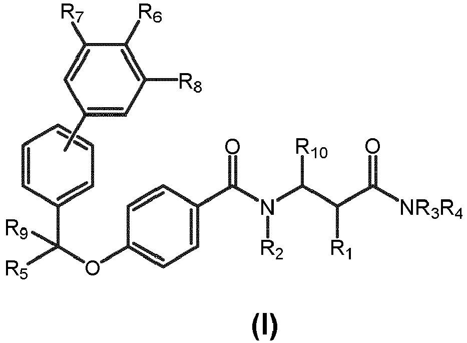 wherein:
**R₁** is a hydrogen atom, a halogen atom, a branched or unbranched C₁₋₆ alkyl radical or - O**R₁ₐ**;
**R₁ₐ** is a hydrogen atom or a branched or unbranched C₁₋₆ alkyl radical;
**R₂** is a hydrogen atom or a branched or unbranched C₁₋₆ alkyl radical;
**R₃** is a hydrogen atom, a branched or unbranched C₁₋₆ alkyl, or -(CH₂)ₘ-O**R₃ₐ;**
**R₃ₐ** is a hydrogen atom or a branched or unbranched C₁₋₆ alkyl radical;
**R₄** is a hydrogen atom, a branched or unbranched C₁₋₆ alkyl, or -(CH₂)ₙ-O**R₄ₐ**;
**R₄ₐ** is a hydrogen atom or a branched or unbranched C₁₋₆ alkyl radical;
**R₅** is a hydrogen atom; a branched or unbranched C₁₋₆ alkyl radical optionally substituted; a halogen atom; a -CN; a C₁₋₆ haloalkyl radical; an optionally substituted -(CH₂)ᵣ-C₃₋₉ cycloalkyl; an optionally substituted -(CH₂)ₛ-C₃₋₉ heterocycloalkyl containing at least one heteroatom selected from N, O or S; an optionally substituted -(CH₂)ⱼ-aryl; an optionally substituted -(CH₂)ᵢ-heteroaryl containing at least one heteroatom selected from N, O or S; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, -OR₅ₐ, -NR₅ₐR_{5b}, NR₅ₐC(O)R_{5b}, -NR₅ₐC(O)NR_{5b}R_{5c}, - -C(O)OR₅ₐ, -C(O)NR₅ₐR_{5b}, -OCH₂CH₂OH, , - C(CH₃)₂OR₅ₐ;
**R₅ₐ, R_{5b}** and **R_{5c}** are independently selected from a hydrogen atom or branched or unbranched C₁₋₆ alkyl radical;
**R₆, R₇** and **R₈** are independently from one another selected from a hydrogen atom, a branched or unbranched C₁₋₆ alkyl radical optionally substituted; a halogen atom; a -CN; a C₁₋₆ haloalkyl radical; an optionally substituted C₃₋₉ cycloalkyl; an optionally substituted C₃₋₉ heterocycloalkyl containing at least one heteroatom selected from N, O or S; an -O-C₁₋₆-alkyl optionally substituted; an -O-C₁₋₆-haloalkyl; an optionally substituted -O-C₃₋₉ cycloalkyl; an optionally substituted -O-C₃₋₉ heterocycloalkyl containing at least one heteroatom selected from N, O or S; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, , -ORₐ, -NRₐR_{b}, NRₐC(O)R_{b}, -C(O)ORₐ, -C(O)NRₐR_{b}, -OCH₂CH₂OH, - C(CH₃)₂ORₐ;
**Rₐ,** and **R_{b}** are independently selected from a hydrogen atom or branched or unbranched C₁₋₆ alkyl radical
**R₉** is a hydrogen atom or a branched or unbranched C₁₋₆ alkyl radical;
**R₁₀** is a hydrogen atom; a branched or unbranched C₁₋₆ alkyl radical optionally substituted; a -CN; a C₁₋₆ haloalkyl radical; an optionally substituted -(CH₂)ᵣ-C₃₋₉ cycloalkyl;; an optionally substituted -(CH₂)ⱼ-aryl; an -O-C₁₋₆-alkyl optionally substituted; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, - -OR₁₀ₐ, -NR₁₀ₐR_{10b}, NR₁₀ₐC(O)R_{10b}, -NR₁₀ₐC(O)NR_{10b}R_{10c}, -C(O)OR₁₀ₐ, -C(O)NR₁₀ₐR_{10b}, -OCH₂CH₂OH, - NR₁₀ₐS(O)₂NR_{10b}R_{10c}, -C(CH₃)₂OR₁₀ₐ;
**R₁₀ₐ, R_{10b}** and **R_{10c}** are independently selected from a hydrogen atom or branched or unbranched C₁₋₆ alkyl radical;
n is 0, 1, 2 or 3;
m is 0, 1, 2 or 3;
r is 0, 1, 2 or 3;
s is 0, 1, 2 or 3;
j is 0, 1, 2 or 3;
i is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

2. A compound according to claim 1, **R₁** is a hydrogen atom or -O**R₁ₐ**.

3. A compound according to any one of claims 1 to 2 wherein **R₅** is a branched or unbranched C₁₋₆ alkyl radical optionally substituted, preferably isobutyl or isopropyl; or one of the following groups:

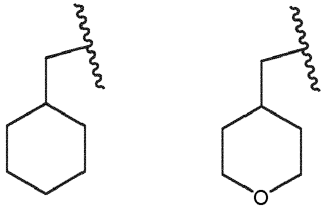

4. A compound according to any one of claims 1 to 3 wherein **R₆**, **R₇** and **R₈** are independently from one another selected from a hydrogen atom, a halogen atom, preferably a F; or a C₁₋₆ haloalkyl radical, preferably a trifluoromethyl.

5. A compound according to any one of claims 1 to 4 wherein **R₁₀** is a hydrogen atom; a branched or unbranched C₁₋₆ alkyl radical optionally substituted, preferably a methyl.

6. A compound according to any one of claims 1 to 5, with the general formula **(Ia)** or (Ib): 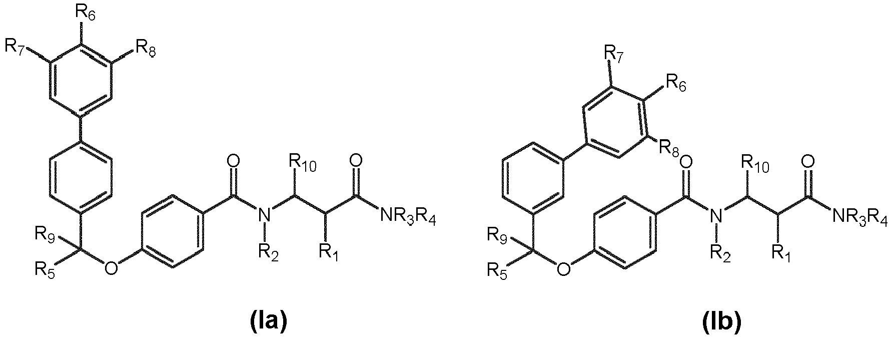 wherein **R₁, R₂, R₃, R₄, R₅, R₆, R₇, R₈, R₉** and **R₁₀** are as defined in any of claims 1 to 5.

7. A compound according to any one of claims 1 to 6, with the general formula **(Ia₁)** or **(Ib₁):** 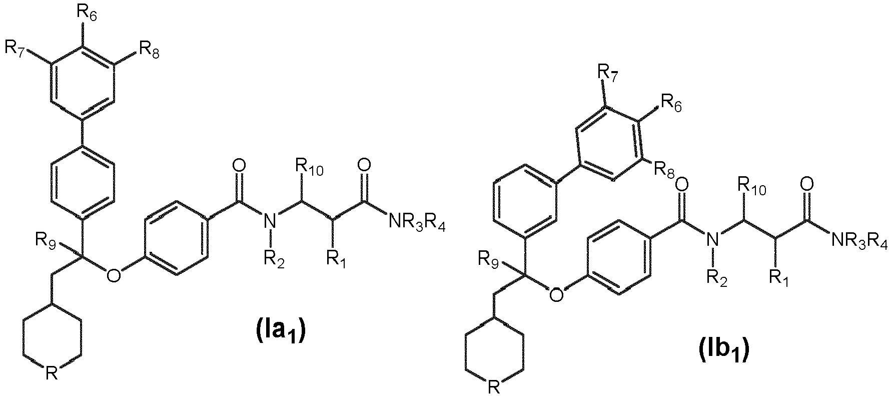 wherein **R₁, R₂, R₃, R₄, R₆, R₇, R₈, R₉** and **R₁₀** are as defined in any of claims 1 to 5 and R represents -CH₂- or -O-.

8. A compound according to claim 1 selected from:
[1] *N*-((S)-3-Amino-2-hydroxy-3-oxopropyl)-4-((S)-2-cyclohexyl-1-(4'-fluoro-[1,1'-biphenyl]-4-yl)ethoxy)benzamide;
**[2]** *N*-((S)-3-Amino-2-hydroxy-3-oxopropyl)-4-((S)-2-cyclohexyl-1-(4'-(trifluoromethyl)-[1,1'-biphenyl]-3-yl)ethoxy)benzamide;
**[3]** *N*-((S)-3-Amino-2-hydroxy-3-oxopropyl)-4-((S)-2-cyclohexyl-1-(4'-(trifluoromethyl)-[1,1'-biphenyl]-4-yl)ethoxy)benzamide;
**[4]** *N*-((S)-3-amino-2-hydroxy-3-oxopropyl)-4-((S)-2-(tetrahydro-2H-pyran-4-yl)-1-(4'-(trifluoromethyl)-[1,1'-biphenyl]-4-yl)ethoxy)benzamide;
**[5]** (*N*-((S)-3-amino-2-hydroxy-3-oxopropyl)-4-((S)-3-methyl-1-(4'-(trifluoromethyl)-[1,1'-bipheny]-4-yl)butoxy)benzamide;
**[6]** *N*-((S)-3-amino-2-hydroxy-3-oxopropyl)-4-((S)-2-methyl-1-(4'-(trifluoromethyl)-[1,1'-biphenyl]-4-yl)propoxy)benzamide;
**[7]** N-((S)-3-amino-2-hydroxy-3-oxopropyl)-4-((R)-2-cyclohexyl-1-(4'-(trifluoromethyl)-[1,1'-biphenyl]-3-yl)ethoxy)benzamide;
[8] 4-((S)-2-cyclohexyl-1-(4'-(trifluoromethyl)-[1,1'-biphenyl]-3-yl)ethoxy)-N-((S)-2-hydroxy-3-(methylamino)-3-oxopropyl)benzamide;
[9] 4-((S)-2-cyclohexyl-1-(4'-(trifluoromethyl)-[1,1'-biphenyl]-3-yl)ethoxy)-N-((S)-3-(dimethylamino)-2-hydroxy-3-oxopropyl)benzamide;
**[10]** N-((R)-3-amino-2-hydroxy-3-oxopropyl)-4-((S)-2-cyclohexyl-1-(4'-(trifluoromethyl)-[1,1'-biphenyl]-3-yl)ethoxy)benzamide;
**[11]** N-((R)-3-amino-2-hydroxy-3-oxopropyl)-4-((R)-2-cyclohexyl-1-(4'-(trifluoromethyl)-[1,1'-biphenyl]-3-yl)ethoxy)benzamide;
**[12]** *N*-((R)-4-amino-4-oxobutan-2-yl)-4-((S)-2-cyclohexyl-1-(4'-(trifluoromethyl)-[1,1'-biphenyl]-4-yl)ethoxy)benzamide;
**[13]** 4-((S)-2-cyclohexyl-1-(4'-(trifluoromethyl)-[1,1'-biphenyl]-3-yl)ethoxy)-N-((S)-2-hydroxy-3-((2-hydroxyethyl)amino)-3-oxopropyl)benzamide; or
**[14]** 4-((S)-2-cyclohexyl-1-(4'-(trifluoromethyl)-[1,1'-biphenyl]-3-yl)ethoxy)-N-((S)-2-hydroxy-3-(hydroxyamino)-3-oxopropyl)benzamide;
or a pharmaceutical acceptable salt, stereoisomer, co-crystal, prodrug or solvate thereof.

9. Process for the preparation of a compound of general formula **(I)**: 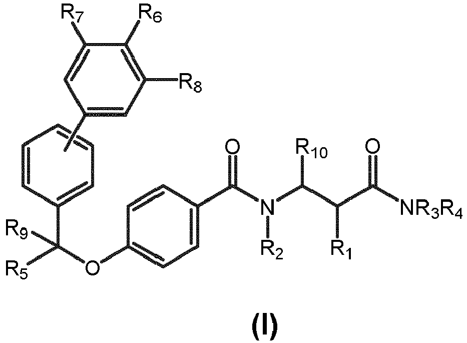
which comprises reacting a compound of formula **(XII)**: 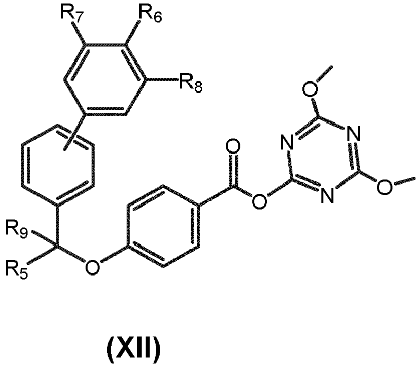
with a compound of formula **(XIII)**: 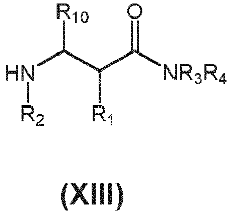
wherein **R₁, R₂, R₃, R₄, R₅, R₆, R₇, R₈, R₉** and **R₁₀** are as defined in any of claims 1 to 5.

10. Process for the preparation of a compound of general formula **(I)**: 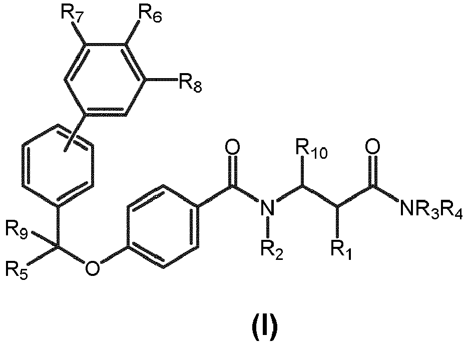
comprising the reaction between a compound of general formula (XVIII): 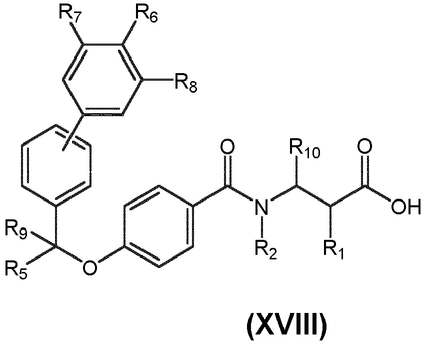
with a compound of formula (XIX): 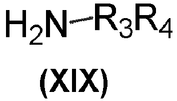
wherein **R₁, R₂, R₃, R₄, R₅, R₆, R₇, R₈, R₉** and **R₁₀** are as defined in any of claims 1 to 5.

11. A compound according to any one of claims 1 to 8 for use as a medicament.

12. A compound according to any one of claims 1 to 8, for use as antibiotic.

13. A compound according to claim 13 for use as antibiotic for the in the treatment and or prophylaxis of *Streptococcus sp.* infections.

14. A compound for use according to claim 13 wherein the infection is an infection caused by *Streptococcus pneumoniae, Streptococcus pyogenes and Streptococcus agalactiae.*

15. A pharmaceutical composition comprising a compound according to any one of claims 1 to 8 or a pharmaceutically acceptable salt, isomer, co-crystal, prodrug or solvate thereof, and at least a pharmaceutically acceptable carrier, additive, adjuvant or vehicle.
